(19) Europäisches Patentamt
European Patent Office
Office européen des brevets

(11) EP 4 450 484 A1

(12) EUROPEAN PATENT APPLICATION
published in accordance with Art. 153(4) EPC

(43) Date of publication:
**23.10.2024 Bulletin 2024/43**

(21) Application number: **22907290.5**

(22) Date of filing: **06.12.2022**

(51) International Patent Classification (IPC):
***C07C 45/50*** *(2006.01)* ***C07C 47/02*** *(2006.01)*
***C07B 61/00*** *(2006.01)*

(52) Cooperative Patent Classification (CPC):
**C07B 61/00; C07C 45/50; C07C 47/02**

(86) International application number:
**PCT/JP2022/044805**

(87) International publication number:
**WO 2023/112762 (22.06.2023 Gazette 2023/25)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **14.12.2021 JP 2021202681**

(71) Applicant: **Mitsubishi Chemical Corporation**
**Tokyo 100-8251 (JP)**

(72) Inventors:
- **MIYAKE, Masashi**
**Tokyo 100-8251 (JP)**
- **SATO, Takashi**
**Tokyo 100-8251 (JP)**

(74) Representative: **Hoffmann Eitle**
**Patent- und Rechtsanwälte PartmbB**
**Arabellastraße 30**
**81925 München (DE)**

# (54) METHOD FOR PRODUCING ALDEHYDE, METHOD FOR PRODUCING ALCOHOL, AND CATALYST COMPOSITION

(57) Provided is a method for producing an aldehyde, comprising causing an olefin to undergo a hydroformylation reaction, in the presence of a catalyst, with a gas which contains hydrogen and carbon monoxide, said method comprising extracting, from a reaction band, part or all of a reaction solution in which a high-boiling-point by-product has been accumulated, mixing a poor solvent for the catalyst into the extracted reaction solution in a mixing tank, precipitating aggregates which contain the catalyst and the high-boiling-point by-product, depositing the precipitated aggregates on the inner surface of the mixing tank, and recovering the aggregates.

*FIG. 1*

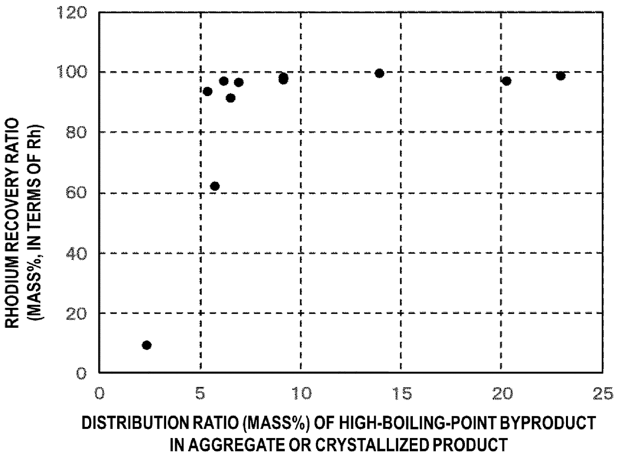


EP 4 450 484 A1

Processed by Luminess, 75001 PARIS (FR)

## Description

### TECHNICAL FIELD

The present invention relates to a method for producing an aldehyde, a method for producing an alcohol, and a catalyst composition.

### BACKGROUND ART

As a method for producing an aldehyde, there is a method for producing an aldehyde by hydroformylating an olefinic unsaturated organic compound with carbon monoxide and hydrogen in the presence of a periodic table Group 8 to 10 metal-phosphine complex catalyst.

The hydroformylation reaction is also called an "oxo reaction". A mixed gas of hydrogen ($H_2$) and carbon monoxide (CO) to be used in the reaction is called an "oxo gas".

The catalyst used for the hydroformylation reaction of an olefin contains an expensive periodic table Group 8 to 10 metal such as rhodium, and it is therefore ideal to use the catalyst semipermanently. Accordingly, a method in which the reaction product is separated from the reaction solution and the reaction solution containing the catalyst as a distillation residue is circulated to a reaction zone and reused, or a method in which the reaction product is distilled off and separated from a reaction zone by using gas stripping and the reaction is continuously performed while allowing the catalyst-containing reaction solution to remain in the reaction zone, is usually employed.

In the hydroformylation reaction, a high-boiling-point byproduct such as an aldehyde condensation byproduct is produced and accumulated, making it necessary to withdraw part of the reaction solution continuously or intermittently outside the reaction zone (the reaction zone may be referred to as a reaction system). Since the withdrawn reaction solution contains a catalyst, in particular, an expensive periodic table Group 8 to 10 metal in addition to the high-boiling-point byproduct, it is extremely important to efficiently recover the catalyst in terms of economy and prevention of environmental pollution.

Patent Literature 1 describes a method in which a hydroformylation reaction solution having accumulated therein a high-boiling-point byproduct and withdrawn from a reaction zone is mixed with an alcohol and water, and the mixture is brought into contact with a hydrogen gas at 30°C, and then cooled to 0°C, thereby crystallizing and recovering a hydrogen-coordinated rhodium-phosphine complex catalyst.

Patent Literature 2 describes a method in which a hydroformylation reaction solution having accumulated therein a high-boiling-point byproduct and withdrawn from a reaction zone is mixed with an alcohol, water, and hydrogen, followed by maintaining at 10°C to 30°C, thereby precipitating and recovering a hydrogen-coordinated rhodium-phosphine complex catalyst.

Patent Literature 3 describes a method in which an alkyl phosphine produced by partially substituting a ligand such as triarylphosphine with an alkyl group of a-olefin is treated with a sufficient amount of oxygen or an oxygen-containing gas at about 20°C to 80°C and converted to its corresponding phosphine oxide and the deactivated catalyst is thereby reactivated.

Patent Literature 4 describes a method in which a hydroformylation reaction solution having accumulated therein a high-boiling-point byproduct and withdrawn from a reaction zone is mixed with an alcohol and water, and the mixture is brought into contact with a hydrogen gas at 30°C, and then cooled to 0°C, thereby crystallizing and recovering a hydrogen-coordinated rhodium-phosphine complex catalyst.

### CITATION LIST

### PATENT LITERATURE

Patent Literature 1: WO2019-098242
Patent Literature 2: JP2006-151826A
Patent Literature 3: JPS57-87845A
Patent Literature 4: JPS57-122948A

## SUMMARY OF INVENTION

### TECHNICAL PROBLEM

In the methods disclosed in Patent Literatures 1 to 4, first, it is necessary to crystallize a rhodium-phosphine complex catalyst in a state in which a high-boiling-point byproduct is removed as much as possible to recover as a crystal having no adhesiveness, and then to separate the crystal of the rhodium-phosphine complex catalyst from a crystallized mother liquid by subjecting the recovered crystal to a filtration treatment. In Patent Literatures 1 to 4, since the loss of the rhodium-phosphine complex catalyst in the filtration treatment is large, the rhodium-phosphine complex catalyst cannot be sufficiently recovered.

An object of the present invention is to solve the problems.

An object of the present invention is to provide a method for producing an aldehyde capable of recovering, from a hydroformylation reaction solution, an expensive complex catalyst, in particular, an expensive periodic table Group 8 to 10 metal in a complex catalyst at a high yield.

An object of the present invention is to provide a method for producing an alcohol in which an aldehyde is produced by the method for producing an aldehyde, followed by producing an alcohol from the aldehyde.

Further, an object of the present invention is to provide a catalyst composition capable of recovering an expensive complex catalyst to be used in a reaction such as a hydroformylation reaction, in particular, an expensive periodic table Group 8 to 10 metal in a complex catalyst at a high yield.

### SOLUTION TO PROBLEM

The present inventors have found that a complex catalyst can be recovered at a yield higher than that in a method of related art by mixing a reaction solution after a hydroformylation reaction with a poor solvent for a catalyst, performing an aggregation treatment in a state of containing the catalyst and a high-boiling-point byproduct, and recovering an aggregate having adhesiveness.

The present invention has been achieved based on such findings, and the gist thereof is as follows.

[1] A method for producing an aldehyde, the method including:

subjecting an olefin to a hydroformylation reaction with a gas containing hydrogen and carbon monoxide in the presence of a catalyst; and
mixing part or all of a reaction solution withdrawn from a reaction zone with a poor solvent for the catalyst to precipitate an aggregate containing the catalyst and having adhesiveness.

[2] The method for producing an aldehyde according to [1], in which
when a total mass of a high-boiling-point byproduct contained in the reaction solution is 100 mass%, a distribution ratio of the high-boiling-point byproduct contained in the aggregate is 4.0 mass% or more.

[3] The method for producing an aldehyde according to [1] or [2], in which
the part or all of the reaction solution is mixed with the poor solvent for the catalyst in a mixing tank, and the precipitated aggregate adheres to an inner surface of the mixing tank, or is settled in an aggregated state in the mixing tank.

[4] A method for producing an aldehyde, the method including:

subjecting an olefin to a hydroformylation reaction with a gas containing hydrogen and carbon monoxide in the presence of a catalyst; and
mixing part or all of a reaction solution withdrawn from a reaction zone with a poor solvent for the catalyst to precipitate an aggregate containing the catalyst, in which
when a total mass of the high-boiling-point byproduct contained in the reaction solution is 100 mass%, a distribution ratio of the high-boiling-point byproduct contained in the aggregate is 4.0 mass% or more.

[5] The method for producing an aldehyde according to [4], in which
the part or all of the reaction solution is mixed with the poor solvent for the catalyst in a mixing tank, and the precipitated aggregate adheres to an inner surface of the mixing tank, or is settled in an aggregated state in the mixing tank.

[6] A method for producing an aldehyde, the method including:

subjecting an olefin to a hydroformylation reaction with a gas containing hydrogen and carbon monoxide in the

presence of a catalyst;
withdrawing part or all of a reaction solution having accumulated therein a high-boiling-point byproduct from a reaction zone;
mixing the withdrawn reaction solution with a poor solvent for the catalyst in a mixing tank to precipitate an aggregate containing the catalyst and the high-boiling-point byproduct; and
adhering the precipitated aggregate to an inner surface of the mixing tank to recover the aggregate.

[7] The method for producing an aldehyde according to [6], in which
the aggregate has adhesiveness under the precipitation condition.
[8] The method for producing an aldehyde according to [6] or [7], in which
when a total mass of the high-boiling-point byproduct contained in the withdrawn reaction solution is 100 mass%, a distribution ratio of the high-boiling-point byproduct in the aggregate is 4.0 mass% or more.
[9] The method for producing an aldehyde according to any one of [1] to [8], in which
the precipitated aggregate is fed to a hydroformylation reaction zone.
[10] The method for producing an aldehyde according to [9], in which
the precipitated aggregate is dissolved in a good solvent for the catalyst and fed to the hydroformylation reaction zone.
[11] The method for producing an aldehyde according to any one of [1] to [10], in which
the precipitation is performed under neutral to acidic conditions.
[12] The method for producing an aldehyde according to any one of [1] to [11], in which
the catalyst is a periodic table Group 8 to 10 metal-organophosphorus complex catalyst.
[13] The method for producing an aldehyde according to [12], in which
the catalyst is a periodic table Group 8 to 10 metal-phosphite complex catalyst.
[14] The method for producing an aldehyde according to [12] or [13], in which
the periodic table Group 8 to 10 metal is rhodium.
[15] The method for producing an aldehyde according to any one of [1] to [14], in which
the poor solvent contains water and an alcohol.
[16] The method for producing an aldehyde according to [15], in which
the poor solvent is a mixture of water and an alcohol, and a content ratio of the water is 12 mass% to 40 mass% with respect to 100 mass% of a total mass of the mixture.
[17] The method for producing an aldehyde according to any one of [1] to [16], in which
the aggregate is precipitated under a condition of a temperature of 0°C or higher and 70°C or lower.
[18] A method for producing an alcohol, the method including:
producing an aldehyde by the method according to any one of [1] to [17], followed by producing an alcohol from the aldehyde.
[19] A catalyst composition, containing:

a catalyst; and
a high-boiling-point byproduct, in which
the catalyst is a periodic table Group 8 to 10 metal-organophosphorus complex catalyst, and
a content ratio of the high-boiling-point byproduct is 30 mass% or more, based on 100% of a total mass of the catalyst composition.

[20] The catalyst composition according to [19], in which
the catalyst composition has adhesiveness.
[21] The catalyst composition according to [19] or [20], in which
the catalyst is a catalyst for producing an aldehyde by subjecting an olefin to a hydroformylation reaction.
[22] The catalyst composition according to any one of [19] to [21], in which
the catalyst is a periodic table Group 8 to 10 metal-phosphite complex catalyst.
[23] The catalyst composition according to any one of [19] to [22], in which
the periodic table Group 8 to 10 metal is rhodium.
[24] A method for producing an aldehyde, the method including:

dissolving the catalyst composition according to any one of [19] to [23] in a good solvent for the catalyst to obtain a catalyst solution; and
subjecting an olefin to a hydroformylation reaction in the presence of the catalyst solution.

ADVANTAGEOUS EFFECTS OF INVENTION

According to the present invention, a method for producing an aldehyde capable of recovering, from a hydroformylation reaction solution, a complex catalyst, in particular, an expensive periodic table Group 8 to 10 metal in a complex catalyst at a high yield can be provided.
According to the present invention, a method for producing an alcohol using an aldehyde produced by the method for producing an aldehyde can also be provided.
Furthermore, according to the present invention, a catalyst composition recovered by the above-described method for producing an aldehyde can be provided.

BRIEF DESCRIPTION OF DRAWINGS

[Fig. 1] Fig. 1 is a graph showing a relationship between a distribution ratio of a high-boiling-point byproduct in an aggregate or a crystallized product and a rhodium recovery ratio in Examples 1 to 10 and Comparative Example 1.
[Fig. 2] (a) of Fig. 2 is an appearance photograph of a mixing tank after aggregation, which is obtained in Example 2, and is an example of a state in which aggregates adhere to an inner surface of the mixing tank and a reaction solution after reaction is solid-liquid separated, and (b) of Fig. 2 is an appearance photograph of a mixing tank after crystallization, which is obtained in Comparative Example 1, and is an example of a state in which a crystallized product does not adhere to an inner surface of the mixing tank and a reaction solution after reaction is not solid-liquid separated and forms a uniform slurry.

DESCRIPTION OF EMBODIMENTS

The present invention is described in detail below. The present invention is not limited to the following description, and can be optionally modified and implemented without departing from the gist of the present invention.
Note that unless otherwise specified, in the present description, a numerical range expressed using "to" means a range that includes numerical values written before and after "to" as an upper limit value and a lower limit value. For example, "A to B" means A or more and B or less.

1. Method for Producing Aldehyde

A first embodiment of a method for producing an aldehyde of the present invention is a method for producing an aldehyde including: subjecting an olefin to a hydroformylation reaction with a gas containing hydrogen and carbon monoxide in the presence of a catalyst (hereinafter, the step may be referred to as a "hydroformylation reaction step"); and mixing part or all of a reaction solution withdrawn from a reaction zone (hereinafter, the step may be referred to as a "reaction solution withdrawing step") with a poor solvent for the catalyst to precipitate an aggregate containing the catalyst and having adhesiveness (hereinafter, the step may be referred to as an "aggregation step").
Furthermore, in the method for producing an aldehyde according to the first embodiment of the present invention, when a total mass of a high-boiling-point byproduct contained in the reaction solution is 100 mass%, a distribution ratio of the high-boiling-point byproduct contained in the aggregate is preferably 4.0 mass% or more.
Furthermore, in the method for producing an aldehyde according to the first embodiment of the present invention, the part or all of the reaction solution is mixed with the poor solvent for the catalyst in a mixing tank, and the precipitated aggregate may adhere to an inner surface of the mixing tank, or may be settled in an aggregated state in the mixing tank.
A second embodiment of the method for producing an aldehyde of the present invention is a method for producing an aldehyde including: subjecting an olefin to a hydroformylation reaction with a gas containing hydrogen and carbon monoxide in the presence of a catalyst (hereinafter, the step may be referred to as a "hydroformylation reaction step"); and mixing part or all of a reaction solution withdrawn from a reaction zone (hereinafter, the step may be referred to as a "reaction solution withdrawing step") with a poor solvent for the catalyst to precipitate an aggregate containing the catalyst (hereinafter, the step may be referred to as an "aggregation step"), in which when a total mass of the high-boiling-point byproduct contained in the reaction solution is 100 mass%, a distribution ratio of the high-boiling-point byproduct contained in the aggregate is preferably 4.0 mass% or more.
Furthermore, in the method for producing an aldehyde according to the second embodiment of the present invention, the part or all of the reaction solution is mixed with the poor solvent for the catalyst in a mixing tank, and the precipitated aggregate may adhere to an inner surface of the mixing tank, or may be settled in an aggregated state in the mixing tank.
A third embodiment of the method for producing an aldehyde of the present invention is a method for producing

an aldehyde including: subjecting an olefin to a hydroformylation reaction with a gas containing hydrogen and carbon monoxide in the presence of a catalyst (hereinafter, the step may be referred to as a "hydroformylation reaction step"); withdrawing part or all of a reaction solution having accumulated therein a high-boiling-point byproduct from a reaction zone (hereinafter, the step may be referred to as a "reaction solution withdrawing step"); mixing the withdrawn reaction solution with a poor solvent for the catalyst in a mixing tank to precipitate an aggregate containing the catalyst and the high-boiling-point byproduct (hereinafter, the step may be referred to as an "aggregation step"); and adhering the precipitated aggregate to an internal surface of the mixing tank and recovering the aggregate (hereinafter, the step may be referred to as a "recovery step").

In the present invention, when the reaction solution withdrawn from a hydroformylation reaction zone is mixed with the poor solvent for the catalyst, an adhesive precipitate containing the catalyst and the high-boiling-point byproduct is precipitated as shown in Examples to be described later. Since the precipitate is adhesive and is not in a crystalline state, the precipitate cannot be sufficiently expressed by the term "crystallized product", and therefore, in the present invention, the precipitate is referred to as an "aggregate" and is distinguished from a non-adhesive precipitate precipitated by a method of related art or in Comparative Example to be described later. On the other hand, the non-adhesive precipitate precipitated by the method of the related art and in Comparative Example to be described later is referred to as a "crystallized product".

As described above, a tank in which the reaction solution and the poor solvent for the catalyst are mixed is referred to as a "mixing tank", "precipitation" of "aggregate" in the "mixing tank" is referred to as an "aggregation treatment", and the step is referred to as an "aggregation step".

On the other hand, as in the method of the related art or Comparative Example to be described later, the "precipitation" of "crystallized product" is referred to as a "crystallization treatment".

In the present description, the "inner surface of the mixing tank" refers to a portion where a flow of the reaction solution is slow in the mixing tank, a portion where the aggregate in the reaction solution is pressed during stirring, or a portion having a shape of a protrusion in the mixing tank, and specific examples thereof include a bottom surface or an inner wall of the mixing tank, a baffle, a stirring blade or a stirring shaft, and a surface of a side pipe.

In the present description, the "reaction zone" and the "hydroformylation reaction zone" refer to a zone including a reactor for performing the hydroformylation reaction and reactor peripheral equipment such as a gas-liquid separator attached to the reactor.

A catalyst composition of the present invention contains: a catalyst; and a high-boiling-point byproduct. The catalyst is a periodic table Group 8 to 10 metal-organophosphorus complex catalyst. A content ratio of the high-boiling-point byproduct is 30 mass% or more, based on 100% of a total mass of the catalyst composition.

First, a hydroformylation reaction according to the first, second, and third embodiments of the method for producing an aldehyde of the present invention (hereinafter may be collectively referred to as "the present invention") will be described, and subsequently, the reaction solution withdrawing step, the aggregation step, and the recovery step will be described for each step.

[Hydroformylation Reaction]

[Catalyst]

In the present invention, the catalyst used for the hydroformylation reaction is not particularly limited as long as it has a catalytic effect on the hydroformylation reaction of an olefin. As the catalyst used for the hydroformylation reaction, it is preferable to use a periodic table Group 8 to 10 metal (hereinafter referred to as "Group 8 to 10 metal")-organophosphorus complex catalyst because of excellent reaction activity thereof.

In the present invention, the Group 8 to 10 metal is a metal belongs to Groups 8 to 10 in the periodic table. Among others, ruthenium, cobalt, rhodium, palladium, and platinum are preferred since they have high activity in case of use as a catalyst, and in particular, rhodium is preferably used since it has high activity.

As an organophosphorus ligand compound for forming the Group 8 to 10 metal-organophosphorus complex catalyst, any trivalent organophosphorus compound that commonly functions as a monodentate ligand or a polydentate ligand for the Group 8 to 10 metal can be used. Among them, examples of the organophosphorus compound serving as a monodentate ligand include a third triorganophosphine represented by the following formula (I).

[Chem. 1]

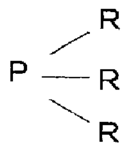

(I)

(In the formula (I), R's each independently represent a substituted or unsubstituted monovalent hydrocarbon group.)

Examples of the monovalent hydrocarbon group represented by R usually include an alkyl group having 1 to 12 carbon atoms, a cycloalkyl group having 3 to 12 carbon atoms, an aryl group having 3 to 12 carbon atoms, an alkylaryl group having 6 to 24 carbon atoms, and an arylalkyl group having 6 to 24 carbon atoms. That is, examples of the above-described triorganophosphine include trialkylphosphine, triarylphosphine, tricycloalkylphosphine, alkylarylphosphine, cycloalkylarylphosphine, and alkylcycloalkylphosphine.

A substituent that the monovalent hydrocarbon group may have is not particularly limited, and examples thereof include an alkyl group and an alkoxy group.

Specific examples of the triorganophosphine include tributylphosphine, trioctylphosphine, triphenylphosphine, tritolylphosphine, tricycloalkylphosphine, monobutyldiphenylphosphine, dipropylphenylphosphine, and cyclohexyldiphenylphosphine. Among them, triphenylphosphine is preferred because of low activity, chemical stability, and easy availability.

As another example of the trivalent organophosphorus compound, for example, a trivalent phosphite compound represented by the following formulae (1) to (10) can be used.

<Trivalent Phosphite Compound Represented by Formula (1)>

[Chem. 2]

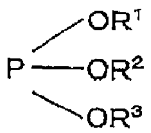

(1)

(In the formula (1), $R^1$ to $R^3$ each independently represent a monovalent hydrocarbon group which may have a substituent.)

Examples of the monovalent hydrocarbon group which may have a substituent and is represented by $R^1$ to $R^3$ include an alkyl group, an aryl group, and a cycloalkyl group.

Specific examples of the compound represented by the formula (1) include trialkyl phosphites such as trimethyl phosphite, triethyl phosphite, n-butyl diethyl phosphite, tri-n-butyl phosphite, tri-n-propyl phosphite, tri-n-octyl phosphite, and tri-n-dodecyl phosphite, triaryl phosphites such as triphenyl phosphite and trinaphthyl phosphite, and alkylaryl phosphites such as dimethylphenyl phosphite, diethylphenyl phosphite, and ethyldiphenyl phosphite. For example, bis(3,6,8-tri-t-butyl-2-naphthyl)phenylphosphite, and bis(3,6,8-tri-t-butyl-2-naphthyl)(4-biphenyl)phenylphosphite described in JPH6-122642A may be used. Among them, triphenyl phosphite is most preferred.

<Trivalent Phosphite Compound Represented by Formula (2)>

[Chem. 3]

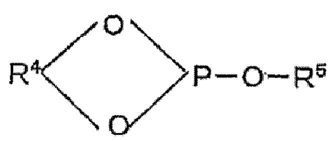

(2)

(In the formula (2), $R^4$ represents a divalent hydrocarbon group which may have a substituent. $R^5$ represents a monovalent

hydrocarbon group which may have a substituent.)

Examples of the divalent hydrocarbon group which may have a substituent for $R^4$ include an alkylene group which may contain oxygen, nitrogen, a sulfur atom, etc. in the middle of a carbon chain, a cycloalkylene group which may contain oxygen, nitrogen, a sulfur atom, etc. in the middle of a carbon chain, a divalent aromatic group such as phenylene and naphthylene, a divalent aromatic group to which a divalent aromatic ring is bonded directly or intermediately through an alkylene group or an atom such as oxygen, nitrogen, or sulfur; and a group to which a divalent aromatic group and an alkylene group are bonded directly or intermediately through an atom such as oxygen, nitrogen, or sulfur.

Examples of the monovalent hydrocarbon group for $R^5$ include an alkyl group, an aryl group, and a cycloalkyl group.

Examples of the compound represented by the formula (2) include compounds described in US Patent No. 3415906 specification, such as neopentyl (2,4,6-t-butyl-phenyl) phosphite, and ethylene (2,4,6-t-butyl-phenyl) phosphite.

<Trivalent Phosphite Compound Represented by Formula (3)>

[Chem. 4]

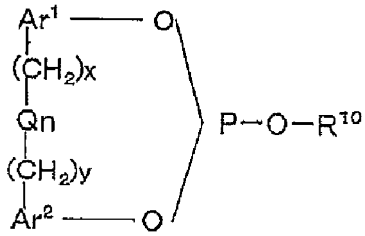

(3)

(In the formula (3), $R^{10}$ has the same meaning as $R^5$ in the above formula (2). Ar' and $Ar^2$ each independently represent an aryl group which may have a substituent. x and y each independently represent 0 or 1. Q is a crosslinking group selected from the group consisting of $-CR^{11}R^{12}-$, -O-, -S-, $-NR^{13}-$, $-SiR^{14}R^{15}$ and -CO-. $R^{11}$ and $R^{12}$ each independently represent a hydrogen atom, an alkyl group having 1 to 12 carbon atoms, a phenyl group, a tolyl group, or an anisyl group. $R^{13}$, $R^{14}$ and $R^{15}$ each independently represent a hydrogen atom or a methyl group. n represents 0 or 1.)

Specific examples of the trivalent phosphite compound represented by the formula (3) include compounds described in US Patent No. 4599206 specification, such as 1,1'-biphenyl-2,2'-diyl-(2,6-di-t-butyl-4-methylphenyl) phosphite, and compounds described in US Patent No. 4717775 specification, such as 3,3'-di-t-butyl-5,5'-dimethoxy-1,1'-biphenyl-2,2'-diyl(2-t-butyl-4-methoxyphenyl) phosphite.

<Trivalent Phosphite Compound Represented by Formula (4)>

[Chem. 5]

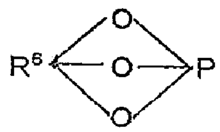

(4)

(In the formula (4), $R^6$ represents a trivalent hydrocarbon group which may have a cyclic or acyclic substituent.)

Examples of the compound represented by the formula (4) include compounds described in US Patent No. 4567306 specification, such as 4-ethyl-2,6,7-trioxa-1-phosphabicyclo-[2,2,2]-octane.

<Trivalent Phosphite Compound Represented by Formulae (5) and (6)>

[Chem. 6]

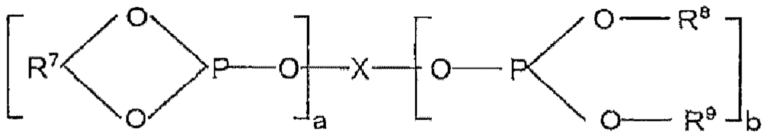

(5)

(In the formula (5), $R^7$ has the same meaning as $R^4$ in the above formula (3). $R^8$ and $R^9$ each independently represent a hydrocarbon group which may have a substituent. a and b each represent an integer of 0 to 6. The sum of a and b is 2 to 6. X represents a (a + b)-valent hydrocarbon group.)

Preferred examples of the compound represented by the formula (5) include a compound represented by the following formula (6). Compounds described in JPS62-116535A and JPS62-116587A are included.

[Chem. 7]

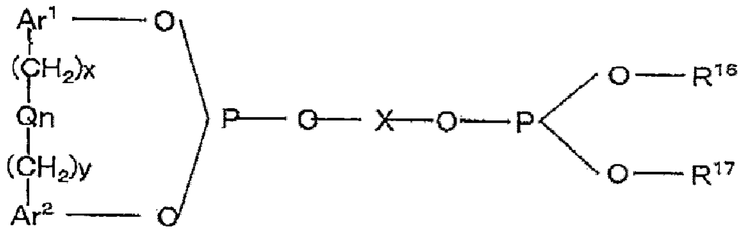

(6)

(In the formula (6), X represents a divalent group selected from the group consisting of alkylene, arylene, and $-Ar^1-(CH_2)x-Qn-(CH_2)y-Ar^2-$. $Ar^1$, $Ar^2$, Q, x, y, and n are the same as $Ar^1$, $Ar^2$, Q, x, y, and n in the above formula (3).)

<Trivalent Phosphite Compound Represented by Formula (7)>

[Chem. 8]

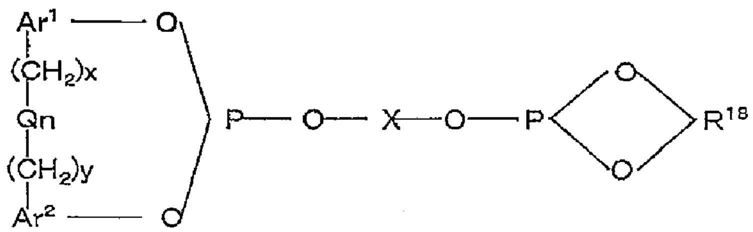

(7)

(In the formula (7), X, $Ar^1$, $Ar^2$, Q, x, y, and n are the same as X, $Ar^1$, $Ar^2$, Q, x, y, and n in the above formula (3). $R^{18}$ has the same meaning as $R^4$ in the above formula (2).)

<Trivalent Phosphite Compound Represented by Formula (8)>

[Chem. 9]

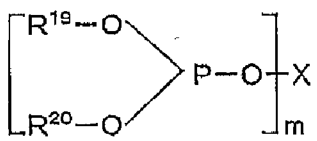

(8)

(In the formula (8), $R^{19}$ and $R^{20}$ each independently represent an aromatic hydrocarbon group, and at least one of the

aromatic hydrocarbon groups has a hydrocarbon group at a carbon atom adjacent to a carbon atom to which an oxygen atom is bonded. m represents an integer of 2 to 4. The -OP($OR^{19}$)($OR^{20}$) groups may be different from each other. X represents an m-valent hydrocarbon group which may have a substituent.)

Among the compound represented by the formula (8), for example, compounds described in JPH5-178779A are preferred.

<Trivalent Phosphite Compound Represented by Formula (9)>

[Chem. 10]

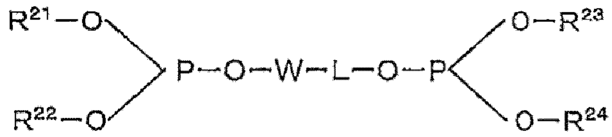

(9)

(In the formula (9), $R^{21}$ to $R^{24}$ each independently represent a hydrocarbon group which may have a substituent. $R^{21}$ and $R^{22}$, and $R^{23}$ and $R^{24}$ may be bonded to each other to form a ring. W represents a divalent aromatic hydrocarbon group which may have a substituent. L represents a saturated or unsaturated divalent aliphatic hydrocarbon group which may have a substituent.)

As the compound represented by the formula (9), for example, those described in JPH8-259578A are used.

<Trivalent Phosphite Compound Represented by Formula (10)>

[Chem. 11]

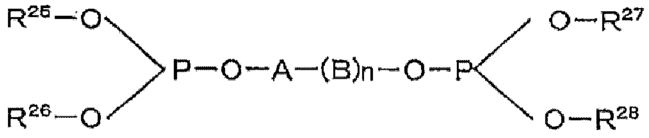

(10)

(In the formula (10), $R^{25}$ to $R^{21}$ each represent a monovalent hydrocarbon group which may have a substituent. $R^{25}$ and $R^{26}$, and $R^{27}$ and $R^{28}$ may be bonded to each other to form a ring. A and B each independently represent a divalent hydrocarbon group which may have a substituent. n represents an integer of 0 or 1.)

Examples of the monovalent hydrocarbon group which may have a substituent and is represented by $R^{25}$ to $R^{28}$ include an alkyl group, an aryl group, and a cycloalkyl group. The divalent hydrocarbon group which may have a substituent for A and B may be any of an aromatic group, an aliphatic group, and a alicyclic group.

<Bisphosphite Compound Represented by Formula (11)>

[Chem. 12]

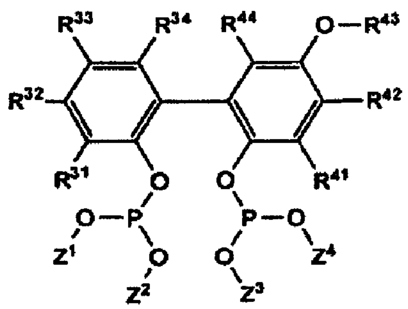

(11)

In the formula (11), $R^{31}$ and $R^{41}$ each independently represent a group selected from the group consisting of a hydrogen atom, an alkyl group having 1 to 20 carbon atoms, and a cycloalkyl group having 3 to 20 carbon atoms.

Examples of the alkyl group having 1 to 20 carbon atoms include a linear or branched alkyl group such as a methyl group, an ethyl group, an n-propyl group, an i-propyl group, an s-butyl group, a t-butyl group, an isopentyl group, a neopentyl group, a t-pentyl group, a t-hexyl group, and a 1,1,2-trimethylpropyl group. Among them, one having 3 to 20 carbon atoms is preferred, one having 4 to 20 carbon atoms is more preferred, and one having 4 to 10 carbon atoms is particularly preferred. Further, one whose carbon atom bonded to an aromatic ring is tertiary is preferred, and examples thereof include a t-butyl group, a t-pentyl group, and a t-hexyl group.

Examples of the cycloalkyl group having 3 to 20 carbon atoms include a cyclohexyl group, a cyclooctyl group, and an adamantyl group. Among them, a cycloalkyl group having 6 to 14 carbon atoms is preferred, and a cycloalkyl group having 6 to 10 carbon atoms is more preferred.

As $R^{31}$ and $R^{41}$, a tertiary alkyl group having 4 to 20 carbon atoms is preferred, a tertiary alkyl group having 4 to 7 carbon atoms is more preferred, and a t-butyl group is particularly preferred. $R^{31}$ and $R^{41}$ may be the same as or different from each other.

When $R^{31}$ and $R^{41}$ are a t-butyl group, a sufficient stabilization effect against hydrolysis of the compound represented by the formula (11) can be obtained due to bulkiness of the t-butyl group. For the above reasons, $R^{31}$ and $R^{41}$ are particularly preferably a t-butyl group.

$R^{32}$ and $R^{42}$ each independently represent a group selected from the group consisting of a hydrogen atom, an alkyl group and an alkoxy group having 1 to 20 carbon atoms, a cycloalkyl group and a cycloalkoxy group having 3 to 20 carbon atoms, a dialkylamino group having 2 to 20 carbon atoms, an aryl group and an aryloxy group having 6 to 20 carbon atoms, an alkylaryl group, an alkylaryoxy group, an arylalkyl group and an arylalkoxy group having 7 to 20 carbon atoms, a cyano group, a hydroxy group, and a halogen atom.

Examples of the alkyl group having 1 to 20 carbon atoms include a linear or branched alkyl group such as a methyl group, an ethyl group, an n-propyl group, an i-propyl group, an n-butyl group, an s-butyl group, a t-butyl group, an n-pentyl group, an isopentyl group, a neopentyl group, a t-pentyl group, and a t-hexyl group.

Examples of the cycloalkyl group having 3 to 20 carbon atoms include a cyclohexyl group, a cyclooctyl group, and an adamantyl group.

Examples of the alkoxy group having 1 to 20 carbon atoms include a methoxy group, an ethoxy group, an isopropoxy group, and a t-butoxy group. Among them, an alkoxy group having 1 to 12 carbon atoms is preferred.

Examples of the cycloalkoxy group having 3 to 20 carbon atoms include a cyclopentyloxy group.

Examples of the dialkylamino group having 2 to 20 carbon atoms include a dimethylamino group and a diethylamino group.

Examples of the aryl group having 6 to 20 carbon atoms include a phenyl group and a naphthyl group.

Examples of the aryloxy group having 6 to 20 carbon atoms include a phenoxy group and a naphthoxy group.

Examples of the alkylaryl group having 7 to 20 carbon atoms include a p-tolyl group and an o-tolyl group.

Examples of the alkylaryoxy group having 7 to 20 carbon atoms include 2,3-xylenoxy.

Examples of the arylalkyl group having 7 to 20 carbon atoms include a benzyl group.

Examples of the arylalkoxy group having 7 to 20 carbon atoms include a 2-(2-naphthyl)ethoxy group.

Examples of the halogen atom include a fluorine atom, a chlorine atom, a bromine atom, and an iodine atom.

$R^{32}$ and $R^{42}$ are preferably a hydrogen atom. A substituent at this position has a small contribution to the effect of improving the reactivity with respect to the hydroformylation reaction and the effect of stabilizing the compound represented by the formula (11) itself. Therefore, from the viewpoint of reducing the production cost of the compound, a hydrogen atom, which is the simplest substituent, is preferred.

$R^{33}$ and $R^{43}$ each independently represent a group selected from the group consisting of a hydrogen atom, an alkyl group having 1 to 20 carbon atoms, a cycloalkyl group having 3 to 20 carbon atoms, an aryl group having 6 to 20 carbon atoms, and an alkylaryl group and an arylalkyl group having 7 to 20 carbon atoms.

Examples of the alkyl group having 1 to 20 carbon atoms include a linear or branched alkyl group such as a methyl group, an ethyl group, an n-propyl group, an i-propyl group, an s-butyl group, a t-butyl group, an isopentyl group, a neopentyl group, a t-pentyl group, and a t-hexyl group. Among them, one having 4 to 20 carbon atoms is preferred, and one having 4 to 10 carbon atoms is particularly preferred. Further, one whose carbon atom bonded to an aromatic ring is tertiary is preferred, and examples thereof include a t-butyl group, a t-pentyl group, and a t-hexyl group.

Examples of the cycloalkyl group having 3 to 20 carbon atoms include a cyclohexyl group, a cyclooctyl group, and an adamantyl group. Among them, a cycloalkyl group having 6 to 14 carbon atoms is preferred, and a cycloalkyl group having 6 to 10 carbon atoms is more preferred.

Examples of the aryl group having 6 to 20 carbon atoms include a phenyl group and a naphthyl group.

Examples of the alkylaryl group having 7 to 20 carbon atoms include a p-tolyl group and an o-tolyl group.

Examples of the arylalkyl group having 7 to 20 carbon atoms include a benzyl group.

$R^{33}$ and $R^{43}$ are each independently preferably a tertiary alkyl group having 4 to 20 carbon atoms, more

preferably a tertiary alkyl group having 4 to 7 carbon atoms, and particularly preferably a t-butyl group.

$R^{34}$ and $R^{44}$ each independently represent a group selected from the group consisting of a hydrogen atom, an alkyl group having 1 to 12 carbon atoms, a cycloalkyl group having 3 to 12 carbon atoms, an alkoxy group having 1 to 12 carbon atoms, a silyl group, a siloxy group, and a halogen atom.

Examples of the alkyl group having 1 to 12 carbon atoms include a linear or branched alkyl group such as a methyl group, an ethyl group, an n-propyl group, an isopropyl group, a t-butyl group, and a decyl group.

Examples of the cycloalkyl group having 3 to 12 carbon atoms include a cyclopropyl group and a cyclohexyl group.

Examples of the alkoxy group having 1 to 12 carbon atoms include a methoxy group, an ethoxy group, and a t-butoxy group.

Examples of the silyl group include a trimethylsilyl group.

Examples of the siloxy group include a silyl group and a trimethylsiloxy group.

Examples of the halogen atom include a fluorine atom, a chlorine atom, a bromine atom, and an iodine atom.

Among them, $R^{34}$ and $R^{44}$ are each independently preferably an alkyl group having 1 to 3 carbon atoms such as a methyl group or an ethyl group, an alkoxy group having 1 to 3 carbon atoms such as a methoxy group or an oxy group, or a halogen atom, and more preferably an alkyl group having 1 to 3 carbon atoms, and $R^{34}$ and $R^{44}$ are particularly preferably a methyl group.

As $R^{34}$ and $R^{44}$, a small alkyl group having 1 to 3 carbon atoms is preferred, and a methyl group is particularly preferred. The reason for this is that the hydroformylation reaction can proceed smoothly and the stability of the compound represented by the formula (11) can be improved at the same time.

$Z^1$ to $Z^4$ are each independently an aryl group having 6 to 20 carbon atoms. The aryl group may have a substituent. Both $Z^1$ and $Z^2$ and $Z^3$ and $Z^4$ are not bonded to each other.

In particular, $Z^1$ to $Z^4$ each independently have no substituent on an aromatic ring carbon atom adjacent to a carbon atom bonded to an oxygen atom, or have a substituent on the aromatic ring carbon atom, and the number of carbon atoms of the substituent is preferably 0 to 2.

When $Z^1$ to $Z^4$ have a substituent on an aromatic ring carbon atom adjacent to a carbon atom bonded to an oxygen atom, the substituent is preferably selected from the group consisting of a group having 1 to 2 carbon atoms such as a methyl group and an ethyl group, a trifluoromethyl group, a cyano group, a nitro group, and a halogen atom such as a chlorine atom and a fluorine atom.

When $Z^1$ to $Z^4$ have a substituent at a position other than the aromatic ring carbon atom, examples of the substituent include: a linear or branched alkyl group having 1 to 12 carbon atoms, and preferably 1 to 8 carbon atoms, such as a methyl group, an ethyl group, an n-propyl group, an i-propyl group, an n-butyl group, an s-butyl group, a t-butyl group, an n-pentyl group, an isopentyl group, a neopentyl group, and a t-pentyl group; an alkoxy group having 1 to 12 carbon atoms, and preferably 1 to 8 carbon atoms, such as a methoxy group and an oxy group; and an aryl group having 6 to 18 carbon atoms, and preferably 6 to 10 carbon atoms, such as a phenyl group and a naphthyl group. Other examples of the substituent include a halogen atom, a cyano group, a nitro group, a trifluoromethyl group, a hydroxyl group, an amino group, an acyl group, a carbonyloxy group, an oxycarbonyl group, an amide group, a sulfonyl group, a sulfinyl group, a silyl group, and a thionyl group. $Z^1$ to $Z^4$ may each have 1 to 5 of the substituents.

Preferred examples of $Z^1$ to $Z^4$ include a phenyl group, a 1-naphthyl group, a 2-naphthyl group, a p-trifluoromethylphenyl group, a 2-ethylphenyl group, a 2-methylphenyl group, a 3-methylphenyl group, a 4-methylphenyl group, a 2,3-dimethylphenyl group, a 2,4-dimethylphenyl group, a 2,5-dimethylphenyl group, a 3,4-dimethylphenyl group, a 3,5-dimethylphenyl group, a 2-chlorophenyl group, a 3-chlorophenyl group, a 4-chlorophenyl group, a 2,3-dichlorophenyl group, a 2,4-dichlorophenyl group, a 2,5-dichlorophenyl group, a 3,4-dichlorophenyl group, a 3,5-dichlorophenyl group, a 2-methoxyphenyl group, a 3-methoxyphenyl group, a 4-methoxyphenyl group, a 2,3-dimethoxyphenyl group, a 3,4-dimethoxyphenyl group, a 3,5-dimethoxyphenyl group, a 4-cyanophenyl group, a 4-nitrophenyl group, a 4-phenylphenyl group, a 5,6,7,8-tetrahydro-1-naphthyl group, a 5,6,7,8-tetrahydro-2-naphthyl group, a 2-methyl-1-naphthyl group, a 4-chloro-1-naphthyl group, a 2-nitro-1-naphthyl group, and a 7-methoxy-2-naphthyl group.

Among them, a 1-naphthyl group or a 2-naphthyl group is preferred from the viewpoint of improving the thermal stability of the ligand and improving the selection ratio for producing a linear aldehyde when producing an aldehyde by a hydroformylation reaction.

The bisphosphite compound represented by the formula (11) is preferably a bisphosphite compound in which $R^{31}$ and $R^{41}$ are each independently a tertiary alkyl group having 4 to 20 carbon atoms, $R^{32}$ and $R^{42}$ are a hydrogen atom, $R^{33}$ and $R^{43}$ are each independently a tertiary alkyl group having 4 to 20 carbon atoms, and $R^{34}$ and $R^{44}$ are each independently selected from the group consisting of an alkyl group having 1 to 3 carbon atoms, an alkoxy group having 1 to 3 carbon atoms, and a halogen atom. Among them, a bisphosphite compound in which $Z^1$ to $Z^4$ each independently have no substituent on an aromatic ring carbon atom adjacent to a carbon atom bonded to an oxygen atom, or have a substituent having 1 to 2 carbon atoms on the aromatic ring carbon atom, and none of $Z^1$ to $Z^4$ are bonded to each other is preferred.

Further, the bisphosphite compound represented by the formula (11) is more preferably a bisphosphite compound in which $R^{31}$, $R^{41}$, $R^{33}$, and $R^{43}$ are each independently a tertiary alkyl group having 4 to 7 carbon atoms, $R^{32}$ and $R^{42}$ each are a hydrogen atom, and $R^{34}$ and $R^{44}$ are each independently an alkyl group having 1 to 3 carbon atoms. Among them, a bisphosphite compound in which $Z^1$ to $Z^4$ are each independently a 1-naphthyl group or a 2-naphthyl group is more preferred, and a bisphosphite compound in which $R^{31}$, $R^{41}$, $R^{33}$, and $R^{43}$ are a t-butyl group and $R^{34}$ and $R^{44}$ are a methyl group is particularly preferred.

Examples of the bisphosphite compound represented by the formula (11) are shown below. The meanings of the symbols in each formula below are as follows.

[Chem. 13]

 : **Methyl group**

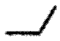 : **Ethyl group**

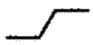 : **n-Propyl group**

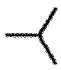 : **i-Propyl group**

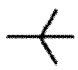 : **t-Butyl group**

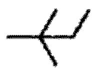 : **t-Pentyl group**

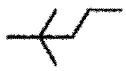 : **t-Hexyl group**

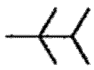 : **1,1,2-Trimethylpropyl group**

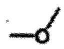 : **Methoxy group**

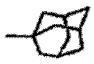 : **Adamantyl group**

[Chem. 14]

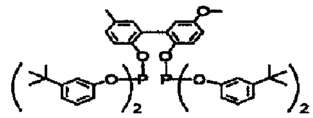

(L-1)

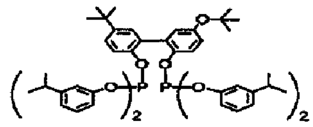

(L-2)

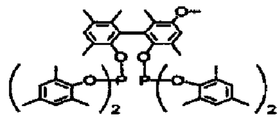

(L-3)

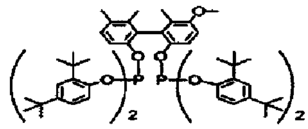

(L-4)

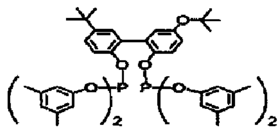

(L-5)

[Chem. 15]

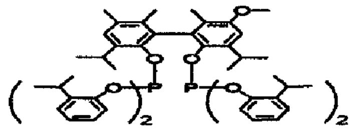

(L-6)

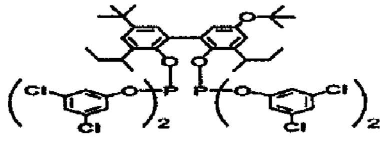

(L-7)

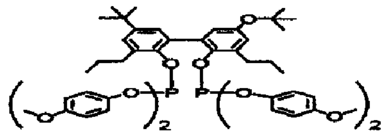

(L-8)

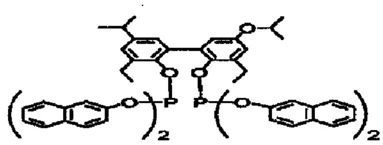

(L-9)

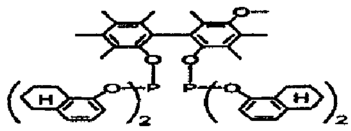

(L-10)

[Chem. 16]

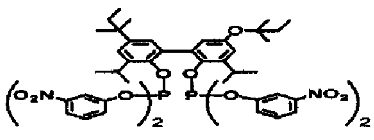

(L-11)

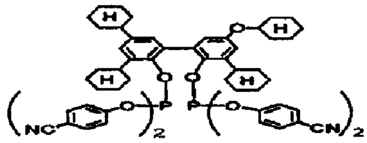

(L-12)

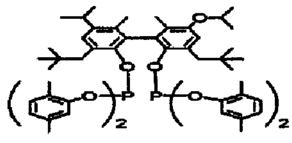

(L-13)

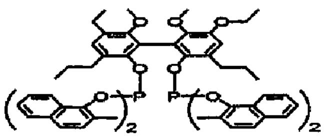

(L-14)

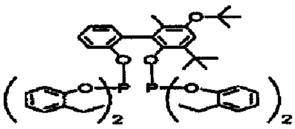

(L-15)

[Chem. 17]

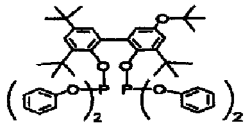

(L-26)

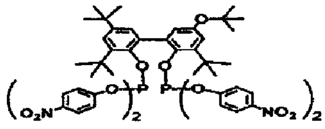

(L-27)

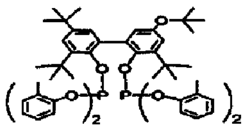

(L-28)

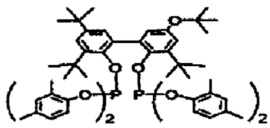

(L-29)

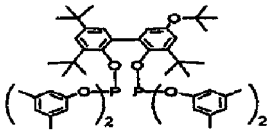

(L-30)

[Chem. 18]

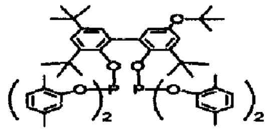

(L-31)

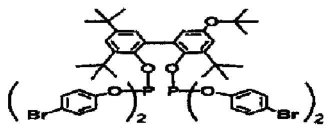

(L-32)

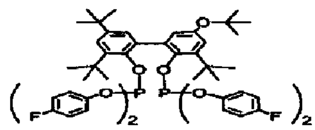

(L-33)

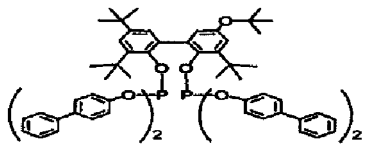

(L-34)

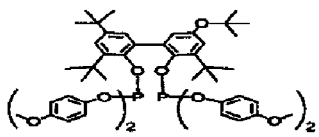

(L-35)

[Chem. 19]

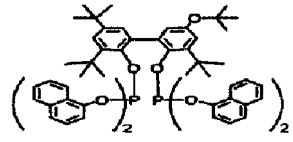

(L-36)

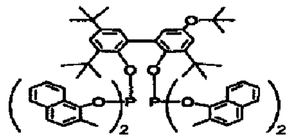

(L-37)

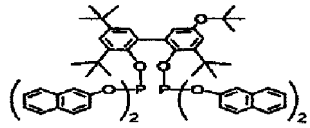

(L-38)

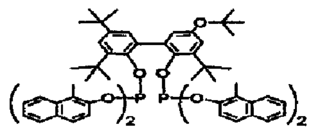

(L-39)

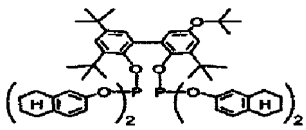

(L-40)

[Chem. 20]

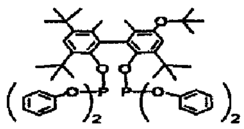

(L-41)

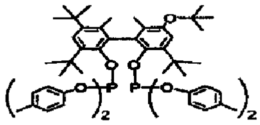

(L-42)

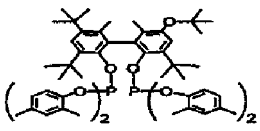

(L-43)

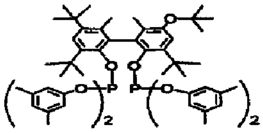

(L-44)

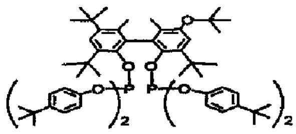

(L-45)

[Chem. 21]

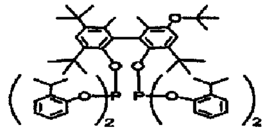

(L-46)

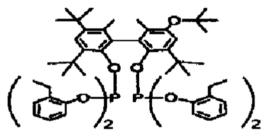

(L-47)

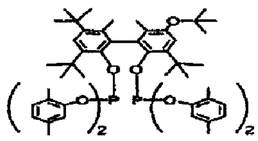

(L-48)

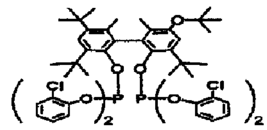

(L-49)

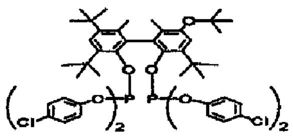

(L-50)

[Chem. 22]

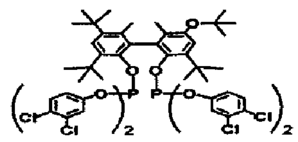

(L-51)

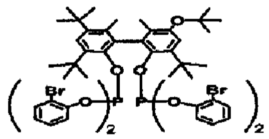

(L-52)

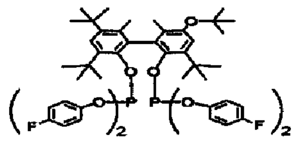

(L-53)

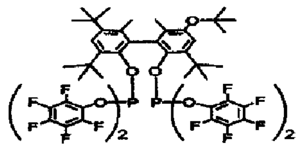

(L-54)

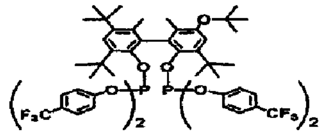

(L-55)

[Chem. 23]

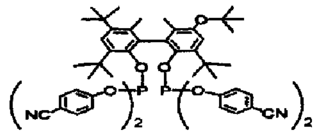

(L-56)

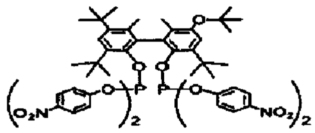

(L-57)

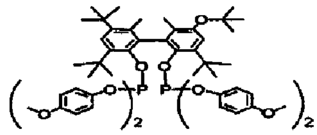

(L-58)

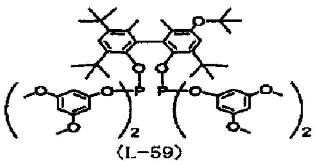

(L-59)

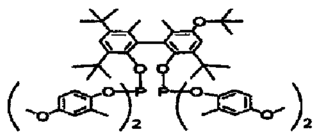

(L-60)

[Chem. 24]

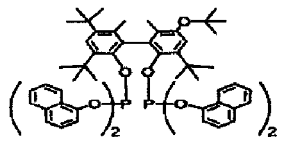

(L-61)

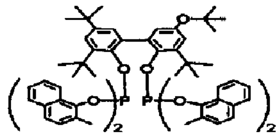

(L-62)

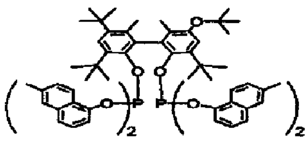

(L-63)

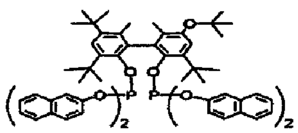

(L-64)

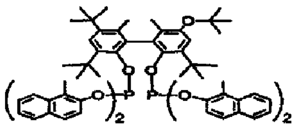

(L-65)

[Chem. 25]

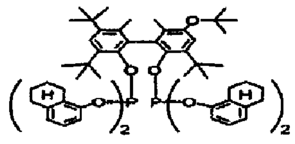

(L-66)

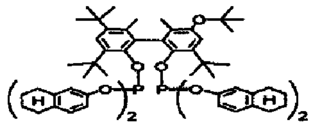

(L-67)

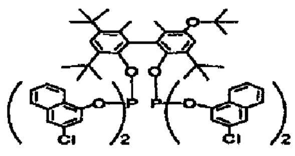

(L-68)

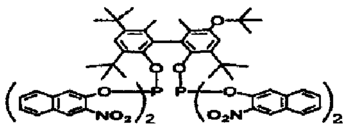

(L-69)

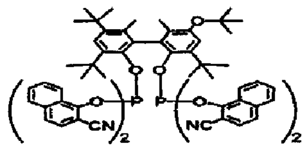

(L-70)

[Chem. 26]

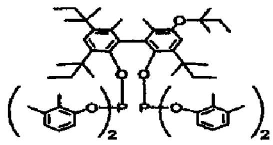

(L-71)

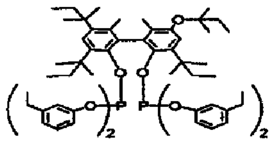

(L-72)

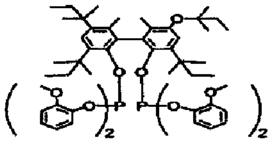

(L-73)

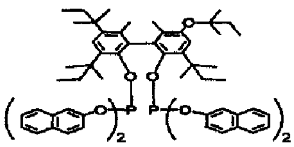

(L-74)

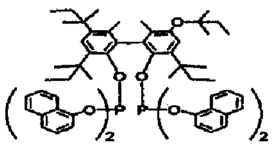

(L-75)

[Chem. 27]

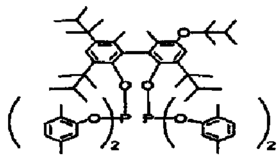

(L-76)

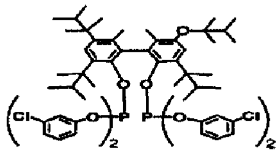

(L-77)

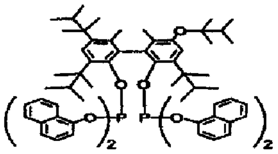

(L-78)

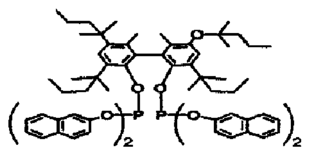

(L-79)

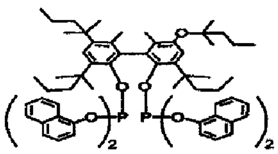

(L-80)

The bisphosphite compound represented by the formula (11) can be produced by a method described in WO2019/039565.

These organophosphorus ligand compounds may be used alone or in combination of two or more thereof, and usually are used alone.

The organophosphorus ligand compound is not particularly limited, and is preferably the phosphite compound represented by the formula (10) or the bisphosphite compound represented by the formula (11), and particularly preferably the bisphosphite compound represented by the formula (11), from the viewpoint of excellent reaction activity and thermal decomposition resistance of the catalyst in the hydroformylation reaction.

That is, the Group 8 to 10 metal-organophosphorus complex catalyst used in the present invention is preferably a Group 8 to 10 metal-phosphite complex catalyst.

The Group 8 to 10 metal-organophosphorus complex catalyst can be more easily prepared by a known complex formation method using a periodic table Group 8 to 10 metal compound (hereinafter, referred to as a "Group 8 to 10 metal compound") and an organophosphorus ligand compound. The complex may be formed in the reaction zone by feeding the Group 8 to 10 metal compound and the organophosphorus ligand compound to the reaction zone. In this case, the organophosphorus ligand compound may be directly introduced into the reaction zone but, considering ease of handling, etc., is preferably introduced after dissolving it in a reaction medium.

The Group 8 to 10 metal compound includes, for example, a water-soluble inorganic salt or inorganic complex compound such as rhodium chloride, palladium chloride, ruthenium chloride, platinum chloride, rhodium bromide, rhodium iodide, rhodium sulfate, rhodium nitrate, palladium nitrate, rhodium ammonium chloride and sodium rhodium chloride; and a water-soluble organic acid salt such as rhodium formate, rhodium acetate, palladium acetate, rhodium propionate, palladium propionate and rhodium octanoate. In addition, respective metal complex species may also be used. Among them, in view of excellent reaction activity and catalyst cost, rhodium acetate is preferably used.

[Hydroformylation Reaction Step]

The hydroformylation reaction is performed by reacting an olefin with hydrogen and carbon monoxide in the presence of a catalyst such as a Group 8 to 10 metal-organophosphorus complex catalyst.
Although a carbon number of olefin is not particularly limited, examples thereof include a carbon number of 2 to 20. The olefin having a carbon number of 2 to 20 may be, for example, an a-olefin such as ethylene, propylene, 1-butene, 1-pentene, 1-hexene and 1-octene, or an internal olefin such as 2-butene, 2-pentene, 3-hexene and 4-octene.
As the reaction medium for the hydroformylation reaction, a solvent that dissolves a raw material olefin and a catalyst such as a Group 8 to 10 metal-organophosphorus complex catalyst, has a boiling point higher than that of an aldehyde to be produced, and does not inhibit the reaction is preferred. Examples of the solvent that can be used in the hydroformylation reaction include: an aromatic hydrocarbon such as benzene, toluene, and xylene; an aliphatic hydrocarbon such as hexane and octane; esters such as butyl acetate and butyl butyrate ester; and ketones.
A concentration of the catalyst in the reaction medium is usually 1 mass ppm to 10 mass% in terms of metal atom of a Group 8 to 10 metal, etc., and the organophosphorus ligand compound such as a phosphite compound used as a ligand is usually present in an excessive amount in the reaction medium in order to increase the stability of the complex catalyst.
The hydroformylation reaction may be performed under known conditions. For example, when among the Group 8 to 10 metal-organophosphorus complex catalyst, in particular, a rhodium-phosphite complex catalyst is used, the reaction conditions are usually appropriately selected within the following ranges.

Hydrogen partial pressure: 0.01 MPaG to 20 MPaG
Carbon monoxide partial pressure: 0.01 MPaGto 20 MPaG
Total pressure: 0.02 MPaG to 30 MPaG
Hydrogen partial pressure/carbon monoxide partial pressure: 0.1 to 10
Reaction temperature: 60°C to 200°C
Rh (rhodium) concentration: several mass ppm to several mass%
P (free organophosphorus ligand)/Rh: 2 to 10000 (molar ratio)
Reaction time: several minutes to several tens of hours

In the hydroformylation reaction, an aldehyde having a carbon number of n+1 can be obtained from a raw material olefin having a carbon number of n (n is, for example, an integer of 2 to 20). Such an aldehyde includes propionaldehyde, butylaldehyde, pentylaldehyde, hexylaldehyde, heptylaldehyde, octylaldehyde, nonylaldehyde, decylaldehyde, etc. Usually, the aldehyde is obtained as a mixture of a linear form and a branched form.
The hydroformylation reaction is performed under the above-described reaction conditions by using usually a flow-type reactor, but a batch-type reactor may also be used.
The main flow reaction (which uses the above flow-type reactor) system includes a stripping system and a liquid circulating system.
The stripping system is a method in which a reaction solution containing a catalyst is held in a reactor, an olefin and an oxo gas are continuously fed, and the aldehyde produced by the reaction is vaporized within the reactor and taken out of the system.
The liquid circulating system is a method in which an olefin, an oxo gas, and a reaction medium containing a catalyst are continuously fed to a reactor and a reaction solution containing the produced aldehyde, the catalyst, the reaction medium, etc. is continuously withdrawn outside the reactor. The reaction solution withdrawn from the reactor is separated into the produced aldehyde and a catalyst-containing reaction solution, for example, by a separation operation such as stripping with an unreacted gas or distillation. The obtained and produced aldehyde is withdrawn outside the system, and the reaction solution containing the catalyst (in the present invention, the reaction solution corresponds to the reaction solution withdrawn in the reaction solution withdrawing step) is usually returned to the reactor and recycled after removing the high-boiling-point byproduct in the reaction solution using a known separation operation such as distillation.
In the present invention, the adhesiveness of the obtained aggregate can be controlled by adjusting the content of the high-boiling-point byproduct in the reaction solution in the aggregation step to be described later.
In the case of the stripping system, a byproduct of the hydroformylation reaction which is a high-boiling-point byproduct accumulates in the catalyst-containing reaction solution held within the reactor. Therefore, usually, part of the reaction solution containing the high-boiling-point byproduct and the catalyst (in the present invention, the reaction solution corresponds to the reaction solution withdrawn in the reaction solution withdrawing step) is intermittently withdrawn outside the reaction zone.
In the case of the liquid circulating system, when recycling of the catalyst-containing reaction solution is continued, a byproduct which is a high-boiling-point byproduct accumulates in the reaction zone. Therefore, usually, part of

the reaction solution containing the high-boiling-point byproduct and the catalyst (in the present invention, the reaction solution corresponds to the reaction solution withdrawn in the reaction solution withdrawing step) is continuously or intermittently withdrawn outside the reaction zone.

The amount of the reaction solution withdrawn from the reaction zone, that is, the reactor may be appropriately determined according to the amount of the high-boiling-point byproduct to be produced.

Usually, when the reaction solution is withdrawn outside the reaction zone, a catalyst in an amount corresponding to the catalyst contained in the withdrawn reaction solution is newly fed to the reaction zone. In the present invention, the amount of catalyst to be newly fed can be reduced by subjecting the withdrawn reaction solution to the aggregation step and returning the reaction solution to the reaction zone. In particular, for the Group 8 to 10 metal, the reaction can be maintained with almost no replenishment.

The constituent component of the high-boiling-point byproduct is various and complicated, but is mainly an aldehyde condensate produced by condensation of an aldehyde which is a target product of the hydroformylation reaction. The aldehyde condensate has a boiling point higher than that of the aldehyde to be produced, and is a high-boiling product that cannot be removed by a simple aldehyde distillation step. Specific examples of such a high-boiling-point byproduct include aldol which is a dimer of the produced aldehyde, an ester compound which is a dimer of the produced aldehyde and is produced by a Tishchenko reaction of the produced aldehyde, an unsaturated aldehyde which is a dehydrated product of the aldol, a saturated aldehyde and a saturated alcohol which are a hydrogenated product of the unsaturated aldehyde, an unsaturated ether which is a dehydrated product of a hemiacetal obtained by a reaction between the produced aldehyde and an alcohol which is a hydrogenated product thereof, an acetal obtained by a reaction between the hemiacetal and the produced aldehyde, and a trimer of the produced aldehyde.

When the Group 8 to 10 metal-phosphite complex catalyst is used as the catalyst, a phosphite or a phosphonate generated by decomposition of a phosphite, and phosphorous acid are present in the reaction solution having accumulated therein the high-boiling-point byproduct.

For example, when rhodium is used as the Group 8 to 10 metal, the following rhodium complexes (a) and (b) are present in the reaction solution having accumulated therein the high-boiling-point byproduct.

(a) A complex in which carbon monoxide and the bisphosphite represented by the formula (11) are coordinated to rhodium (Rh): for example, $RhH(CO)_2$ (bisphosphite) or RhH(CO) (bisphosphite)

(b) A rhodium cluster complex in which a plurality of rhodium atoms are connected and carbon monoxide and the bisphosphite represented by the formula (11) are coordinated thereto: for example, $[Rh(CO)\ (bisphosphite)]_2$ or $[Rh(CO)_2\ (bisphosphite)]_2$

Although rhodium is used as an example in the above, the same can be said for other Group 8 to 10 metals.

[Reaction Solution Withdrawing Step]

The reaction solution withdrawing step is a step of withdrawing part or all of the reaction solution having accumulated therein the high-boiling-point byproduct from the hydroformylation reaction zone, and specifically, a step of withdrawing the reaction solution containing the aldehyde generated by the hydroformylation reaction, the catalyst, and the reaction medium outside the reactor while performing the hydroformylation reaction as described above.

The reaction solution in the hydroformylation reaction zone contains about 40 mass% to 80 mass% of the high-boiling-point byproduct. A poor solvent for the catalyst is added to and mixed with the reaction solution containing the high-boiling-point byproduct in the aggregation step to crystallize an aggregate containing the catalyst and the high-boiling-point byproduct. Accordingly, as disclosed in the above-mentioned Patent Literatures 1 to 4, as compared with a method of separating a crystal of a catalyst from a crystallized mother liquid by performing crystallization in a state where the high-boiling-point byproduct is removed as much as possible and subjecting the precipitated crystal to a filtration treatment, the loss of the catalyst in the filtration treatment can be eliminated, and the catalyst can be recovered at a high recovery ratio.

In the reaction solution withdrawing step, if necessary, the hydroformylation reaction solution withdrawn from the hydroformylation reaction zone may be subjected to the aggregation step after removing a light-boiling-point component in the reaction solution. In order to remove the light-boiling-point component from the reaction solution, known separation operations such as distillation can be used.

When the rhodium-phosphite complex catalyst is used as the catalyst, the reaction solution withdrawn in the reaction solution withdrawing step, that is, the reaction solution before an aggregation treatment, usually has the following composition, and the reaction solution is subjected to the following aggregation step.

(Composition of Reaction Solution Withdrawn in Reaction Solution Withdrawing Step)

Rhodium atoms in complex catalyst: 50 mass ppm to 2000 mass ppm
Phosphite compound: 1000 mass ppm to 3 mass%
n-Aldehyde: 1 mass% to 30 mass%
Others (various complexes, high-boiling-point byproducts, etc.): 40 mass% to 80 mass%

The "various complexes" contained in the "other components" refer to the various rhodium complexes contained in the reaction solution having accumulated therein the high-boiling-point byproduct, as listed in the section of [Hydroformylation Reaction Step] above.

[Aggregation Step]

In the aggregation step, the poor solvent for the catalyst (hereinafter, may be simply referred to as a "poor solvent") is added to and mixed with the reaction solution withdrawn in the reaction solution withdrawing step to precipitate an aggregate containing the catalyst and the high-boiling-point byproduct.

Specifically, the withdrawn reaction solution and the poor solvent are put into a mixing tank to precipitate an aggregate.

The poor solvent is a solvent in which the Group 8 to 10 metal compound has smaller solubility than in the reaction solution. The poor solvent is preferably a solvent that keeps a homogeneous phase with the reaction solution and does not participate in the reaction in the reaction zone.

Specific examples of the poor solvent include methanol, ethanol, propanol (n-, i-), butanol (n-, i-, t), acetone, and a mixture of acetone and water.

From the viewpoint of the recovery ratio of the Group 8 to 10 metal-phosphite complex catalyst, a mixture of water and an alcohol is preferred, and a mixture of water and an alcohol having 1 to 3 carbon atoms is particularly preferred. A content ratio of water in the mixture is preferably 12 mass% to 40 mass%, more preferably 13 mass% to 25 mass%, and still more preferably 15 mass% to 22 mass% with respect to 100 mass% of the mixture. When the content ratio of water is equal to or more than the lower limit, the recovery ratio of the complex catalyst is increased due to the solubility of the complex. When the content ratio of water is equal to or less than the upper limit, the reaction solution tends to become a homogeneous phase, and the recovery ratio of the complex catalyst can be satisfactorily maintained.

A mixing ratio (a mass ratio) of the poor solvent and the reaction solution is preferably 5: 1 to 15: 1, more preferably 6:1 to 10:1, and still more preferably 7:1 to 9:1, depending on the type of the poor solvent and the composition of the reaction solution. When the ratio of the poor solvent is equal to or less than the upper limit, the amount of the complex catalyst dissolved in the poor solvent decreases, the recovery ratio of the catalyst increases, and an aggregate recovery device can be reduced in size. When the ratio of the poor solvent is equal to or more than the lower limit, the reaction solution tends to become a homogeneous phase, and the recovery ratio of the complex catalyst can be satisfactorily maintained.

The withdrawn reaction solution may be directly mixed with the poor solvent or may be mixed with the poor solvent after removing at least part of the reaction medium by distillation, etc.

The reaction solution and the poor solvent are preferably mixed under stirring.

A temperature in the aggregation step is preferably 0°C to 70°C, more preferably 0°C to 40°C, and further preferably 5°C to 20°C. When the temperature during aggregation is equal to or lower than the upper limit, the recovery ratio of the catalyst is excellent. When the temperature during the aggregation is equal to or higher than the lower limit, the poor solvent is maintained in a liquid state, and the cooling energy can be prevented.

An aggregation time is not particularly limited. The aggregation time is usually 10 minutes to 10 hours, and more preferably 30 minutes to 5 hours.

The aggregation step is preferably performed in an inert gas atmosphere such as nitrogen gas or argon gas, from the viewpoint of preventing oxidation and deactivation of the complex catalyst contained in the reaction solution and the aggregate.

The aggregation step is preferably performed by stirring the reaction solution and the poor solvent under neutral to acidic conditions, since it is not necessary to perform an operation such as a washing or neutralization treatment when the solution containing the aggregate after the aggregation is fed to the hydroformylation reaction zone. Usually, the reaction solution withdrawn from the hydroformylation reaction zone is weakly acidic, and a solution obtained by mixing a poor solvent containing a mixed solution of water and alcohol with the reaction solution has a pH of about 4 to 7. Therefore, the aggregation operation can be performed without performing any particular pH adjustment.

By stirring the reaction solution and the poor solvent for a predetermined time in the above preferred temperature range in an inert gas atmosphere, an aggregate is precipitated. Therefore, as the mixing tank used in the aggregation step, a glass container or stainless steel container, and a SUS container with a stirrer, which can be sealed, are preferably used.

The aggregate precipitated by mixing the reaction solution withdrawn from the hydroformylation reaction zone with the poor solvent has adhesiveness under aggregation conditions. Here, the expression "having adhesiveness" means that the precipitated aggregate has an adhesive force sufficient to adhere to an inner surface of the mixing tank in the aggregation step of the present invention. Since the aggregate has adhesiveness as described above, the precipitated aggregate is separated from the solution in the mixing tank, and due to the adhesiveness, the aggregate adheres to the inner surface of the mixing tank, that is, a bottom surface or an inner wall of the mixing tank, a stirring blade, etc.

That is, in the present invention, since the reaction solution containing the high-boiling-point byproduct is withdrawn from the hydroformylation reaction zone to perform aggregation, the aggregate precipitated by the aggregation contains the complex catalyst and the high-boiling-point byproduct, and the presence of the high-boiling-point byproduct allows the aggregate to have adhesiveness. By incorporating the complex catalyst into the aggregate having adhesiveness, the complex catalyst can be recovered at a high recovery ratio.

In the production method of the present invention, in order to precipitate the aggregate having adhesiveness as described above, cause the precipitated aggregate to adhere to the inner surface of the mixing tank, and recover the aggregate to increase the recovery ratio of the complex catalyst, it is preferable to perform the aggregation under such a condition that the high-boiling-point byproduct is transferred to the aggregate at a certain distribution ratio. Specifically, when a total mass of the high-boiling-point byproduct contained in the reaction solution withdrawn from the hydroformylation reaction zone is 100 mass%, the lower limit of the distribution ratio of the high-boiling-point byproduct in the aggregate is preferably 4.0 mass% or more, more preferably 5.0 mass% or more, and further preferably 6.0 mass% or more. On the other hand, when the distribution ratio of the high-boiling-point byproduct in the aggregate is excessively large, the yield of the aggregate decreases, and therefore the aggregation is preferably performed such that the upper limit of the distribution ratio of the high-boiling-point byproduct in the aggregate is usually 30 mass% or less, more preferably 20 mass% or less, and further preferably 10 mass% or less.

The upper limit and the lower limit can be freely combined. That is, the distribution ratio of the high-boiling-point byproduct in the aggregate is preferably 4.0 mass% or more and 30 mass% or less, more preferably 5.0 mass% or more and 20 mass% or less, and still more preferably 6.0 mass% or more and 10 mass% or less, when the total mass of the high-boiling-point byproduct contained in the reaction solution withdrawn from the hydroformylation reaction zone is 100 mass%.

The distribution ratio can be controlled by adjusting aggregation conditions such as the composition of the poor solvent, the ratio of the poor solvent and the reaction solution, and the aggregation temperature.

Details of a method for measuring the "distribution ratio" will be described later in the section of Examples.

[Recovery Step]

In the recovery step, the aggregate precipitated in the aggregation step and adhering to the inner surface of the mixing tank is recovered. The recovered aggregate is preferably fed to the hydroformylation reaction zone.

The method for recovering the aggregate is not particularly limited, and it is preferable to recover the aggregate by dissolving the aggregate in a good solvent for the complex catalyst in the aggregate, since the operation is simple and the recovery ratio of the complex catalyst can be increased. Specifically, the solution in the mixing tank is withdrawn to leave only the aggregate, and the good solvent for the complex catalyst is added thereto to dissolve the aggregate.

The good solvent for the complex catalyst used in this case is not particularly limited. The good solvent is preferably those described above as a reaction medium used in the hydroformylation reaction or an aldehyde, which is a product obtained by the hydroformylation reaction, since the aggregate solution can be directly fed to the hydroformylation reaction zone.

Specific examples of the good solvent include, when the raw material olefin is propylene, aldehydes such as n-butyl aldehyde and i-butyl aldehyde, and aromatic hydrocarbons such as benzene, toluene, and xylene.

An amount of the good solvent to be used for dissolving the aggregate is not particularly limited, and it is preferable that the amount of the good solvent used to dissolve the aggregate is smaller, from the viewpoint of reducing the volume of the mixing tank. The amount of the good solvent varies depending on the solubility of the catalyst with respect to the good solvent to be used and the content of the high-boiling-point compound in the aggregate, and the amount of the good solvent is generally preferably about 5 times to 100 times by mass with respect to the aggregate.

A temperature for dissolving the aggregate in the good solvent is preferably about 5°C to 60°C.

The aggregate solution can be directly fed to the hydroformylation reaction zone, and if necessary, unnecessary components may be removed by a known separation operation such as distillation, or a solvent or a complex catalyst having high catalytic activity may be added and then fed to the hydroformylation reaction zone.

In the present invention, such a reaction solution containing the complex catalyst and the high-boiling-point byproduct is withdrawn from the hydroformylation reaction zone, and the poor solvent is added and mixed in the mixing tank to precipitate the adhesive aggregate containing the complex catalyst and the high-boiling-point byproduct, and the precipitated aggregate adheres to the inner surface of the mixing tank and is recovered. By using such a method, as

compared with a method of the related art in which a complex catalyst is precipitated as a crystal having no adhesiveness in a state where a high-boiling-point byproduct is removed as much as possible and a slurry in which a crystallized product is uniformly dispersed in a mother liquid is obtained, as in a crystallization method of the related art, in the production method of the present invention, the loss of the complex catalyst due to the filtering operation or the like of the slurry can be eliminated and the complex catalyst can be recovered at a high recovery ratio and reused. Therefore, in the production method of the present invention, the expensive Group 8 to 10 metal such as rhodium in the complex catalyst cannot be wasted, and the productivity can be improved.

2. Method for Producing Alcohol

In the method for producing an alcohol of the present invention, an aldehyde, which is produced by the method for producing an aldehyde of the present invention is used.

An alcohol can be produced by directly reacting an aldehyde with hydrogen, namely hydrogenation reaction, or bringing into hydrogenation reaction after dimerization.

In the hydrogenation reaction, known solid catalysts in which metals such as nickel, chromium, and copper are supported in a carrier can be used. Reaction conditions thereof are usually a temperature of 60°C to 200°C and a hydrogen pressure of 0.1 MPaG to 20 MPaG.

3. Catalyst Composition

The catalyst composition of the present invention contains: a catalyst; and a high-boiling-point byproduct. The catalyst is a periodic table Group 8 to 10 metal-organophosphorus complex catalyst. A content ratio of the high-boiling-point byproduct is 30 mass% or more, based on 100% of a total mass of the catalyst composition.

When the catalyst composition of the present invention satisfies the above configuration, the catalyst composition has adhesiveness, and the catalyst composition is recovered by adhering the catalyst composition to an inner surface of a reaction device such as a mixing tank, thereby improving the recovery ratio of the catalyst.

The method for obtaining the catalyst composition of the present invention is not particularly limited, and examples thereof include a method for obtaining an aggregate according to any of the methods of the first, second, and third embodiments of the method for producing an aldehyde of the present invention described above.

In the catalyst composition of the present invention, a catalyst same as the catalyst used in the first, second, and third embodiments of the method for producing an aldehyde of the present invention described above can be adopted as the catalyst.

In the catalyst composition of the present invention, a high-boiling-point byproduct same as those described in the first, second, and third embodiments of the method for producing an aldehyde of the present invention can be adopted as the high-boiling-point byproduct.

In the catalyst composition of the present invention, a catalyst same as the periodic table Group 8 to 10 metal-organophosphorus complex catalyst described in the first, second, and third embodiments of the method for producing an aldehyde of the present invention can be adopted as the periodic table Group 8 to 10 metal-organophosphorus complex catalyst.

In the catalyst composition of the present invention, a lower limit of the content ratio of the high-boiling-point byproduct is 30 mass% or more, preferably 40 mass% or more, more preferably 50 mass% or more, further preferably 60 mass% or more, and particularly preferably 70 mass% or more, with respect to 100% of the total mass of the catalyst composition, from the viewpoint that the catalyst composition has adhesiveness, and the catalyst composition is recovered by adhering the catalyst composition to the inner surface of the reaction device such as a mixing tank, thereby increasing the recovery ratio of the catalyst such as the complex catalyst. On the other hand, an upper limit of the content ratio of the high-boiling-point byproduct is not particularly limited, and the content ratio may be preferably 97 mass% or less, more preferably 90 mass% or less, and further preferably 80 mass% or less, with respect to 100% of the total mass of the catalyst composition, from the viewpoint that the catalyst composition is easily precipitated as an aggregate.

In the catalyst composition of the present invention, a lower limit of the content ratio of the catalyst is not particularly limited, and the content ratio may be preferably 3 mass% or more, more preferably 10 mass% or more, and further preferably 20 mass% or more, with respect to 100% of the total mass of the catalyst composition, from the viewpoint that the catalyst composition is easily precipitated as an aggregate. On the other hand, an upper limit of the content ratio of the catalyst is preferably 70 mass% or less, more preferably 60 mass% or less, still more preferably 50 mass% or less, further preferably 40 mass% or less, and particularly preferably 30 mass% or less, with respect to 100% of the total mass of the catalyst composition, from the viewpoint that the medium composition has adhesiveness, and the catalyst composition is recovered by adhering the catalyst composition to the inner surface of the reaction device such as a mixing tank, thereby increasing the recovery ratio of the catalyst such as the complex catalyst.

The catalyst composition of the present invention may contain other components such as a reaction solvent

and an aldehyde, which is a target product of the hydroformylation reaction, in a content ratio of 10 mass% or less, and preferably 5 mass% or less with respect to the 100% of total mass of the catalyst composition, in addition to the high-boiling-point byproduct and the catalyst.

4. Method for Producing Aldehyde

The method for producing an aldehyde of the present invention is a method for producing an aldehyde including dissolving the catalyst composition of the present invention in a good solvent for the catalyst to obtain a catalyst solution, and subjecting an olefin to a hydroformylation reaction in the presence of the catalyst solution to obtain an aldehyde corresponding to the olefin.
A method for dissolving the catalyst composition of the present invention in a good solvent for the catalyst to obtain a catalyst solution is not particularly limited. For example, a method and conditions same as those of the method for obtaining an aggregate solution using the catalyst composition of the present invention in place of an aggregate in the recovery step of the method according to any of the first, second, and third embodiments of the method for producing an aldehyde of the present invention described above can be adopted.
The method for subjecting an olefin to a hydroformylation reaction in the presence of the catalyst solution to obtain an aldehyde corresponding to the olefin is not particularly limited. For example, conditions same as those exemplified in the hydroformylation reaction step in the method according to any of the first, second, and third embodiments of the method for producing an aldehyde of the present invention can be adopted.

Examples

Hereinafter, the present invention will be described in more detail with reference to Examples. The present invention is not limited to the following Examples as long as the scope of the present invention is not exceeded.
Compounds used in Examples and Comparative Examples are as follows.

Rhodium acetate aqueous solution (rhodium acetate concentration: 28 mass%) (trade name: rhodium acetate L, manufactured by N.E. CHEMCAT CORPORATION)
n-Butyl aldehyde (purity: 99% or more, manufactured by Mitsubishi Chemical Corporation)
Metanol (trade name: special grade reagent, Metanol, manufactured by FUJIFILM Wako Pure Chemical Corporation)
Desalted water (manufactured by Mitsubishi Chemical Corporation)

Phosphite ligand A (bisphosphite ligand compound shown below, synthesized by a method described in WO2019/039565)

[Chem. 28]

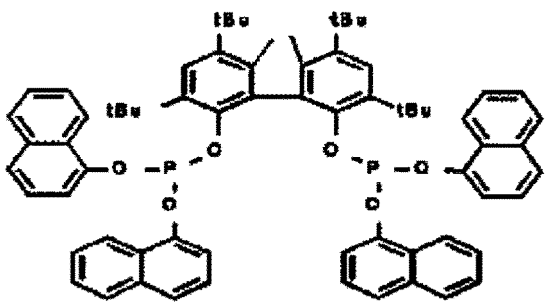

Phosphite ligand A

(1,5-Cyclooctadiene)acetate rhodium dimer (trade name: $Rh_2(cod)_2(OAc)_2$, manufactured by N.E. CHEMCAT CORPORATION)

[Example 1]

A hydroformylation reaction of propylene was performed using rhodium acetate as a periodic table Group 8 to 10 metal compound, and a phosphite ligand A as an organophosphorus ligand. Next, a reaction solution was withdrawn from a hydroformylation reaction zone, and a light-boiling-point component was distilled off using a distillation method.

A composition of the reaction solution after distillation and before being subjected to an aggregation treatment was as follows.

An amount of rhodium atoms in the complex catalyst in the reaction solution was determined by X-ray fluorescence analysis. An amount of n-butyl aldehyde was determined by a gas chromatography internal standard method. An amount of the phosphite ligand A was determined by a high-performance liquid chromatography external standard method.

(Composition of Reaction Solution Before Aggregation Treatment)

Rhodium atoms in complex catalyst: 373 mass ppm
Phosphite ligand A: 12006 mass ppm
n-Butyraldehyde: 10.6 mass%
Others (various complexes, high-boiling-point byproducts, etc.): 88.2 mass%
A glass container with an electromagnetic induction stirrer having a volume of 0.5 L was used as a mixing tank.

The withdrawn reaction solution and a mixed solvent of methanol and water (methanol:water = 80:20 (mass ratio)) as a poor solvent were put into the mixing tank in an inert gas (nitrogen gas) atmosphere at a ratio such that a mass ratio of poor solvent/reaction solution was 9.00. After the mixing tank was sealed, the mixture was kept at a temperature of 10°C for 2 hours while being stirred at 500 rpm to perform the aggregation treatment, thereby precipitating a rhodium complex together with a high-boiling-point byproduct.

(Form of Aggregation Treatment Solution and Property of Aggregate)

When the aggregation treatment solution was visually observed, the precipitated aggregate adhered to an inner wall of the mixing tank, a stirring blade, etc., thereby solid-liquid separating the aggregate and the mother liquid. The adhering aggregate had a property of an adhesive substance.

(Distribution Ratio of High-boiling-point Byproduct in Aggregate)

Thereafter, the mother liquid was withdrawn from the mixing tank, and then 400 mL of n-butyl aldehyde was introduced into the mixing tank to dissolve the aggregate adhering to the inside of the mixing tank at room temperature, and the aggregate solution was recovered.

A content M1 (unit: mg) of the high-boiling-point byproduct contained in the aggregate solution and a content M2 (unit: mg) of the high-boiling-point byproduct contained in the mother liquid withdrawn from the mixing tank were measured by the gas chromatography internal standard method, and a distribution ratio of the high-boiling-point byproduct in the aggregate was calculated using the following formula. As a result, the distribution ratio was 9.2 mass%.

$$\text{Distribution ratio (mass\%) of high-boiling-point byproduct} = M1/(M1 + M2) \times 100$$

(Rhodium Recovery Ratio)

As the recovery ratio of the complex catalyst, the rhodium recovery ratio was measured according to the following procedure.

An amount R1 (in terms of rhodium atom, unit: mg) of the rhodium complex catalyst contained in the aggregate solution and an amount R2 (in terms of rhodium atom, unit: mg) of the rhodium complex catalyst contained in the mother liquid withdrawn from the mixing tank were measured by X-ray fluorescence analysis, and the rhodium recovery ratio (in terms of rhodium atom, mass%) was calculated using the following formula. As a result, the recovery ratio was 96.6 mass%.

$$\text{Rhodium recovery ratio (mass\%)} = R1/(R1 + R2) \times 100$$

(Production of Aldehyde)

The aggregate solution and toluene as a solvent were mixed, and 54 ml of the obtained mixed solution was

put into an upper and lower stirring type autoclave having a volume of 0.2 L in a nitrogen atmosphere, and the autoclave was sealed. The composition of the reaction solution before the hydroformylation reaction was as follows.

(Composition of Reaction Solution before Hydroformylation Reaction)

Rhodium atoms in complex catalyst: 152 mass ppm
Phosphite ligand A: 5929 mass ppm
Toluene: 45.0 mass%
Others (various complexes, high-boiling-point byproducts, etc.): 54.4 mass%

Next, 3.6 g of propylene was fed to an autoclave, and the temperature was increased to 70°C. Thereafter, a mixed gas of hydrogen and carbon monoxide (hydrogen:carbon monoxide =1:1 (volume ratio)) was injected thereto such that the total pressure in the autoclave was 1.0 MPaA, and a hydroformylation reaction was performed for 2 hours while maintaining the pressure and the temperature. An apparent reaction rate (a rate constant) of the hydroformylation reaction, which was calculated based on a propylene conversion ratio, a propane selection ratio, an aldehyde selection ratio, an N/I ratio (a ratio of n-butyl aldehyde to i-butyl aldehyde) of a target product butyraldehyde, a half-life of propylene, and a reduction ratio of carbon monoxide during the hydroformylation reaction when the hydroformylation reaction of propylene was performed, was determined. The evaluation results are shown in Table 2.

[Reference Example 1]

For comparison with an aldehyde production experiment in Example 1, an aldehyde production experiment was performed by the following procedure using a commercially available rhodium catalyst.

As a commercially available rhodium catalyst, a (1,5-cyclooctadiene)acetate rhodium dimer (trade name: $Rh_2(cod)_2(OAc)_2$, manufactured by N.E. CHEMCAT CORPORATION) was used.

$Rh_2(cod)_2(OAc)_2$, a bisphosphite ligand A, and toluene as a solvent were mixed, 54 mL of the obtained mixed solution was put in an upper and lower stirring type autoclave having a volume of 0.2 L in a nitrogen atmosphere, and the autoclave was sealed. The composition of the reaction solution before the hydroformylation reaction is shown in Table 2.

Next, an aldehyde was produced under the same conditions as in Example 1 except that the reaction conditions shown in Table 2 were used. The evaluation results are shown in Table 2.

[Examples 2 to 10]

An aggregation treatment was performed under the same conditions as in Example 1 except that a composition of a reaction solution before the aggregation treatment or a composition of a poor solvent (water/methanol) was changed as shown in Table 1, thereby obtaining an aggregate containing a rhodium complex catalyst. Further, an aldehyde was produced using the obtained aggregate under the same conditions as in Example 1. The evaluation results are shown in Table 1.

An appearance photograph of the mixing tank after aggregation, which was obtained in Example 2, was shown in (a) of Fig. 2. As a result of visual observation, an aggregate adhering to the inside of the mixing tank and the reaction solution after the reaction exhibited a solid-liquid separation state.

[Comparative Example 1]

A crystallization treatment was performed under the same conditions as in the aggregation treatment in Example 1 except that a composition of a reaction solution before the crystallization treatment and a composition of a poor solvent (water/methanol) were changed as shown in Table 1, and a crystallized product containing a rhodium complex catalyst was obtained.

(Form of Crystallization Treatment Solution and Property of Crystallized Product)

An appearance photograph of the mixing tank after the crystallization treatment, which was obtained in Comparative Example 1, was shown in (b) of Fig. 2. As a result of visual observation, the crystallization treatment solution was not solid-liquid separated and formed a uniform slurry. The adhesion of the crystallized product to the inside of the mixing tank was not confirmed.

(Distribution Ratio of High-boiling-point Byproduct in Crystallized Product)

Thereafter, the obtained slurry was solid-liquid separated using a commercially available polytetrafluoroethylene membrane filter having a hole diameter of 0.5 μm. In the same manner as in Example 1, the amount R1 (in terms of rhodium atom, unit: mg) of the rhodium complex catalyst contained in the solution of the crystallized product and the amount R2 (in terms of rhodium atom, unit: mg) of the rhodium complex catalyst in the recovered crystallized product were measured by a fluorescent X-ray analysis method, and the rhodium recovery ratio was calculated using the following formula, and as a result, the recovery ratio was 9.3 mass%.

$$\text{Rhodium recovery ratio (mass\%)} = R1/(R1 + R2) \times 100$$

A relationship between the distribution ratio of the high-boiling-point byproduct in the aggregate in Examples 1 to 10 and the crystallized product in Comparative Example 1 and the rhodium recovery ratio was shown in Fig. 1.

[Table 1]

Table 1

| | | Unit | Example 1 | Example 2 | Example 3 | Example 4 | Example 5 |
|---|---|---|---|---|---|---|---|
| Solution composition of reaction solution | Rhodium atoms in complex catalyst | Mass ppm | 373 | 379 | 367 | 386 | 386 |
| | Phosphite ligand in complex catalyst | Mass ppm | 12006 | 12749 | 10130 | 12007 | 12007 |
| | n-Butyraldehyde | Mass% | 10.6 | 10.1 | 10.9 | 9.9 | 99 |
| Aggregation or crystallization conditions | Aggregation or crystallization temperature | °C | 10 | 10 | 10 | 10 | 10 |
| | Aggregation or crystallization time | hr | 120 | 120 | 120 | 120 | 120 |
| | Composition of poor solvent (water/ methanol) | Mass% | 20/80 | 20/80 | 20/80 | 20/80 | 15/85 |
| | Mass ratio of poor solvent/reaction solution | - | 9.00 | 9.00 | 9.00 | 9.00 | 9.00 |
| | Distribution ratio of high-boiling-point byproduct in aggregate or crystallized product | Mass% | 5.4 | 6.2 | 9.1 | 9.2 | 5.7 |
| | Form of aggregation treatment solution or crystallization treatment solution (visual observation) | - | Solid-liquid separation | Solid-liquid separation | Solid-liquid separation | Solid-liquid separation | Solid-liquid separation |
| | Property of aggregate or crystallized product | - | Adhesive substance | Adhesive substance | Adhesive substance | Adhesive substance | Adhesive substance |
| Results | Rhodium recovery ratio | Mass% (in terms of Rh) | 93.8 | 97.0 | 97.4 | 98.2 | 62.7 |
| | Content of high-boiling-point byproduct in aggregate or crystallized product | Mass% | 56.9 | 57.4 | 72.2 | 94.4 | 73.5 |

table 1 (continued)

| | | Unit | Example | | | | | Comparative Example |
|---|---|---|---|---|---|---|---|---|
| | | | 6 | 7 | 8 | 9 | 10 | 1 |
| Solution composition of reaction solution | Rhodium atoms in complex catalyst | Mass ppm | 400 | 345 | 372 | 372 | 381 | 345 |
| | Phosphite ligand in complex catalyst | Mass ppm | 11753 | 9547 | 10514 | 10514 | 9943 | 9547 |
| | n-Butyraldehyde | Mass% | 9.9 | 100 | 10.2 | 10.2 | 8.6 | 100 |
| Aggregation or crystallization conditions | Aggregation or crystallization temperature | °C | 10 | 10 | 10 | 10 | 10 | 10 |
| | Aggregation or crystallization time | hr | 120 | 120 | 120 | 120 | 120 | 120 |
| | Composition of poor solvent (water/methanol) | Mass% | 20/80 | 25/75 | 30/70 | 41/59 | 20/80 | 10/90 |
| | Mass ratio of poor solvent/reaction solution | - | 9.00 | 9.00 | 9.00 | 9.00 | 9.00 | 9.00 |
| | Distribution ratio of high-boiling-point byproduct in aggregate or crystallized product | Mass% | 6.5 | 13.9 | 22.9 | 20.3 | 6.9 | 2.3 |
| | Form of aggregation treatment solution or crystallization treatment solution (visual observation) | - | Solid-liquid separation | Solid-liquid separation | Solid-liquid separation | Solid-liquid separation | Solid-liquid separation | Uniform slurry |
| | Property of aggregate or crystallized product | - | Adhesive substance | Adhesive substance | Adhesive substance | Adhesive substance | Adhesive substance | Crystal (no adhesiveness) |
| Results | Rhodium recovery ratio | Mass% (in terms of Rh) | 91.4 | 99.7 | 98.6 | 97.0 | 96.6 | 9.3 |
| | Content of high-boiling-point byproduct in aggregate or crystallized product | Mass% | 62.0 | 88.7 | 64.5 | 35.6 | 54.6 | 25.3 |

[Table 2]

| | | Unit | Example 1 | Reference Example 1 |
|---|---|---|---|---|
| Solution composition of reaction solution | Rhodium atoms in complex catalyst | Mass ppm | 152 | 138 |
| | Phosphite ligand A in complex catalyst | Mass ppm | 5929 | 6454 |
| | Toluene | Mass% | 45.0 | 45.6 |
| | Others (various complexes, high-boiling-point byproducts, etc.) | Mass% | 54.4 | 53.8 |
| Reaction conditions | Reaction temperature | °C | 70 | 70 |
| | Total pressure | MPaA | 1.0 | 10 |
| | Initial oxo gas partial pressure | MPaA | 0.50 | 0.48 |
| | Ratio of hydrogen/carbon monoxide | Volume ratio | 1/1 | 1/1 |
| | Reaction time | hr | 2.5 | 2.5 |
| Results | Propylene conversion ratio | % | 96.3 | 96.4 |
| | Propane selection ratio | % | 1.1 | 1.2 |
| | Aldehyde selection ratio | % | 98.9 | 98.8 |
| | N/I ratio | - | 78 | 80 |
| | Half-life of propylene | min | 14.0 | 15.0 |
| | Apparent rate constant | 1/hr | 2.97 | 2.77 |

The above results show the following.

In Examples 1 to 10, the distribution ratio of the high-boiling-point byproduct in the aggregate was significantly increased and the aggregation conditions were controlled such that the aggregate having adhesiveness was precipitated, so that the solid-liquid separation proceeded after the aggregation treatment, and almost all of the precipitated aggregates adhered to the mixing tank, specifically, the stirring blades and the introduction pipe in the glass container. Further, by dissolving and recovering the adhesive product from the mixing tank, the rhodium complex catalyst could be recovered at a high yield.

From the results shown in Table 2, the rhodium complex catalyst recovered in Example 1 had a catalytic performance equivalent to that of a complex catalyst using a commercially available rhodium catalyst in the production of aldehyde.

On the other hand, in Comparative Example 1, the distribution ratio of the high-boiling-point byproduct in the crystallized product was low, so that the obtained crystallized product was crystallized as a crystal having no adhesiveness. The form of the crystallization treatment solution was a uniform slurry state. When the crystallized product was recovered using a filtration method, the recovery ratio of the rhodium complex catalyst was low.

While the present invention has been described in detail and with reference to specific embodiments thereof, it will be apparent to one skilled in the art that various changes and modifications can be made therein without departing from the spirit and scope of the present invention.

The present application is based on a Japanese patent application filed on December 14, 2021 (patent application No. 2021-202681), which is hereby incorporated by reference in its entirety.

## Claims

**1.** A method for producing an aldehyde, the method comprising:

subjecting an olefin to a hydroformylation reaction with a gas containing hydrogen and carbon monoxide in the presence of a catalyst; and

mixing part or all of a reaction solution withdrawn from a reaction zone with a poor solvent for the catalyst to precipitate an aggregate containing the catalyst and having adhesiveness.

2. The method for producing an aldehyde according to claim 1, wherein
when a total mass of a high-boiling-point byproduct contained in the reaction solution is 100 mass%, a distribution ratio of the high-boiling-point byproduct contained in the aggregate is 4.0 mass% or more.

3. The method for producing an aldehyde according to claim 1 or 2, wherein
the part or all of the reaction solution is mixed with the poor solvent for the catalyst in a mixing tank, and the precipitated aggregate adheres to an inner surface of the mixing tank, or is settled in an aggregated state in the mixing tank.

4. A method for producing an aldehyde, the method comprising:

subjecting an olefin to a hydroformylation reaction with a gas containing hydrogen and carbon monoxide in the presence of a catalyst; and
mixing part or all of a reaction solution withdrawn from a reaction zone with a poor solvent for the catalyst to precipitate an aggregate containing the catalyst, wherein
when a total mass of the high-boiling-point byproduct contained in the reaction solution is 100 mass%, a distribution ratio of the high-boiling-point byproduct contained in the aggregate is 4.0 mass% or more.

5. The method for producing an aldehyde according to claim 4, wherein
the part or all of the reaction solution is mixed with the poor solvent for the catalyst in a mixing tank, and the precipitated aggregate adheres to an inner surface of the mixing tank, or is settled in an aggregated state in the mixing tank.

6. A method for producing an aldehyde, the method comprising:

subjecting an olefin to a hydroformylation reaction with a gas containing hydrogen and carbon monoxide in the presence of a catalyst;
withdrawing part or all of a reaction solution having accumulated therein a high-boiling-point byproduct from a reaction zone;
mixing the withdrawn reaction solution with a poor solvent for the catalyst in a mixing tank to precipitate an aggregate containing the catalyst and the high-boiling-point byproduct; and
adhering the precipitated aggregate to an inner surface of the mixing tank to recover the aggregate.

7. The method for producing an aldehyde according to claim 6, wherein
the aggregate has adhesiveness under the precipitation condition.

8. The method for producing an aldehyde according to claim 6 or 7, wherein
when a total mass of the high-boiling-point byproduct contained in the withdrawn reaction solution is 100 mass%, a distribution ratio of the high-boiling-point byproduct in the aggregate is 4.0 mass% or more.

9. The method for producing an aldehyde according to any one of claims 1 to 8, wherein
the precipitated aggregate is fed to a hydroformylation reaction zone.

10. The method for producing an aldehyde according to claim 9, wherein
the precipitated aggregate is dissolved in a good solvent for the catalyst and fed to the hydroformylation reaction zone.

11. The method for producing an aldehyde according to any one of claims 1 to 10, wherein
the precipitation is performed under neutral to acidic conditions.

12. The method for producing an aldehyde according to any one of claims 1 to 11, wherein
the catalyst is a periodic table Group 8 to 10 metal-organophosphorus complex catalyst.

13. The method for producing an aldehyde according to claim 12, wherein
the catalyst is a periodic table Group 8 to 10 metal-phosphite complex catalyst.

**14.** The method for producing an aldehyde according to claim 12 or 13, wherein the periodic table Group 8 to 10 metal is rhodium.

**15.** The method for producing an aldehyde according to any one of claims 1 to 14, wherein the poor solvent contains water and an alcohol.

**16.** The method for producing an aldehyde according to claim 15, wherein the poor solvent is a mixture of water and an alcohol, and a content ratio of the water is 12 mass% to 40 mass% with respect to 100 mass% of a total mass of the mixture.

**17.** The method for producing an aldehyde according to any one of claims 1 to 16, wherein the aggregate is precipitated under a condition of a temperature of 0°C or higher and 70°C or lower.

**18.** A method for producing an alcohol, the method comprising: producing an aldehyde by the method according to any one of claims 1 to 17, followed by producing an alcohol from the aldehyde.

**19.** A catalyst composition comprising:

a catalyst; and
a high-boiling-point byproduct, wherein
the catalyst is a periodic table Group 8 to 10 metal-organophosphorus complex catalyst, and
a content ratio of the high-boiling-point byproduct is 30 mass% or more, based on 100% of a total mass of the catalyst composition.

**20.** The catalyst composition according to claim 19, wherein the catalyst composition has adhesiveness.

**21.** The catalyst composition according to claim 19 or 20, wherein the catalyst is a catalyst for producing an aldehyde by subjecting an olefin to a hydroformylation reaction.

**22.** The catalyst composition according to any one of claims 19 to 21, wherein the catalyst is a periodic table Group 8 to 10 metal-phosphite complex catalyst.

**23.** The catalyst composition according to any one of claims 19 to 22, wherein the periodic table Group 8 to 10 metal is rhodium.

**24.** A method for producing an aldehyde, the method comprising:

dissolving the catalyst composition according to any one of claims 19 to 23 in a good solvent for the catalyst to obtain a catalyst solution; and
subjecting an olefin to a hydroformylation reaction in the presence of the catalyst solution.

FIG. 1

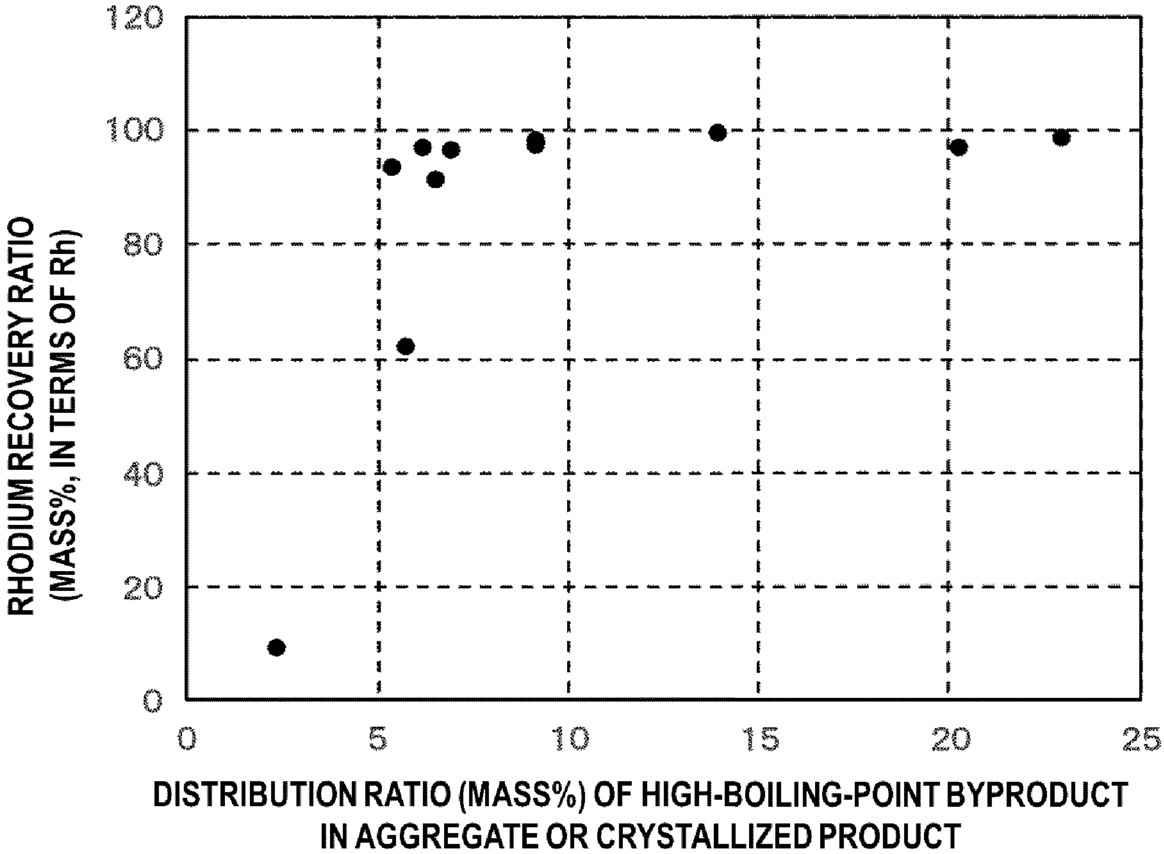
120
100
80
60
40
20
0
0
5
10
15
20
25
RHODIUM RECOVERY RATIO
(MASS%, IN TERMS OF Rh)
DISTRIBUTION RATIO (MASS%) OF HIGH-BOILING-POINT BYPRODUCT
IN AGGREGATE OR CRYSTALLIZED PRODUCT

FIG. 2

(a)

(b)

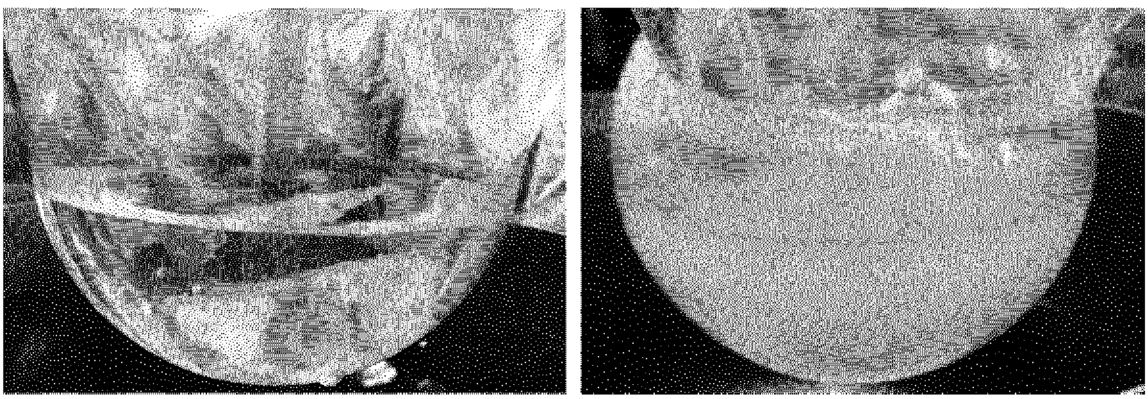

## INTERNATIONAL SEARCH REPORT

International application No.
**PCT/JP2022/044805**

**A. CLASSIFICATION OF SUBJECT MATTER**

***C07C 45/50***(2006.01)i; ***C07C 47/02***(2006.01)i; ***C07B 61/00***(2006.01)i
FI: C07C45/50; C07C47/02; C07B61/00 300

According to International Patent Classification (IPC) or to both national classification and IPC

**B. FIELDS SEARCHED**

Minimum documentation searched (classification system followed by classification symbols)

C07C45/50; C07C47/02; C07B61/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | WO 2019/098242 A1 (MITSUBISHI CHEMICAL CORPORATION) 23 May 2019 (2019-05-23)<br>claims, examples | 1-8, 11-23 |
| Y | claims, paragraph [0050], examples | 9-10, 24 |
| X | JP 57-122948 A (MITSUBISHI KASEI KOGYO KK) 31 July 1982 (1982-07-31)<br>claims, examples | 1-8, 11-23 |
| Y | claims, p. 5, upper left column, line 16 to upper right column, line 16, examples, reference examples | 9-10, 24 |
| X | JP 2001-114794 A (MITSUBISHI CHEMICAL CORPORATION) 24 April 2001 (2001-04-24)<br>claims, examples | 1-8, 11-23 |
| Y | claims, paragraphs [0084]-[0085], reference examples, examples | 9-10, 24 |
| X | JP 2006-151890 A (MITSUBISHI CHEMICAL CORPORATION) 15 June 2006 (2006-06-15)<br>claims, examples | 1-8, 11-23 |
| Y | claims, paragraph [0048], examples | 9-10, 24 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 February 2023** | **14 February 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/044805**

**C. DOCUMENTS CONSIDERED TO BE RELEVANT**

| Category* | Citation of document, with indication, where appropriate, of the relevant passages | Relevant to claim No. |
|---|---|---|
| X | JP 55-159841 A (UNION CARBIDE CORP) 12 December 1980 (1980-12-12) claims, examples | 19-24 |
| Y | claims, p. 14, upper right column, line 10 to lower left column, line 2, examples | 9-10, 24 |
| X | WO 2005/049202 A1 (MITSUBISHI CHEMICAL CORPORATION) 02 June 2005 (2005-06-02) claims, examples | 19, 21-24 |
| Y | claims, p. 10, line 18 to p. 11, line 1, examples | 9-10, 24 |
| X | JP 8-337550 A (MITSUBISHI CHEMICAL CORPORATION) 24 December 1996 (1996-12-24) claims, paragraphs [0107]-[0110] | 19, 21-24 |
| Y | claims, paragraphs [0100], [0107]-[0110] | 9-10, 24 |
| A | JP 2013-147445 A (MITSUBISHI CHEMICAL CORPORATION) 01 August 2013 (2013-08-01) claims, paragraphs [0006]-[0008], examples, comparative examples | 1-24 |

Form PCT/ISA/210 (second sheet) (January 2015)

INTERNATIONAL SEARCH REPORT

International application No.
PCT/JP2022/044805

**Box No. III Observations where unity of invention is lacking (Continuation of item 3 of first sheet)**

This International Searching Authority found multiple inventions in this international application, as follows:

(Invention 1) Claims 1-18
Documents 1-4 disclose "a method for producing aldehyde, the method including reacting an olefin and a gas containing hydrogen and carbon monoxideto for hydroformylation in the presence of a catalyst, the method further including: mixing a poor solvent of the catalyst with some or all of a reaction solution extracted from a reaction band, and precipitating an adhesive aggregate that contains the catalyst and a high boiling point by-product" (document 1: claims, examples; document 2: claims, examples; document 3: claims, examples; document 4: claims). Claims 1-8 lack novelty in the light of documents 1-4 and thus do not have special technical features. However, claims 9-10 depending from claims 1, 4, and 6 have the special technical feature of "the precipitated aggregate is supplied to the hydroformylation reaction band", and the sections of claims 11-18 citing claims 9-10 have the same special technical feature as this. Therefore, the aforementioned sections of claims 1-18 are classified as invention 1.
Furthermore, the sections other than the aforementioned sections of claims 1-8 and 11-18 depend from claims 1, 4, and 6 and are inventively linked to claims 1, 4, and 6, and thus are classified as invention 1.

(Invention 2) Claims 19-24
Claims 19-24 share, with invention 1, the feature of a "catalyst composition containing a catalyst". However, this feature does not make a contribution over the prior art in the light of the content disclosed in documents 1-7 (see above in regard to documents 1-4; document 5: claims, examples; document 6: claims, examples; document 7: claims, paragraphs [0107]-[0110]), and thus cannot be said to be a special technical feature. Furthermore, there is no other identical or corresponding special technical feature between claims 19-24 and invention 1.
Additionally, claims 19-24 do not depend from claims 1, 4, and 6. Moreover, claims 19-24 are not substantially identical to or similarly closely related to any of the claims classified as invention 1.
Therefore, claims 19-24 cannot be classified as invention 1.
The inventions in claims 19-24 are classified as invention 2 as a result of having the special technical feature of "a catalyst composition containing a catalyst and a high boiling point byproduct, wherein the catalyst is a long-period periodic table group 8-10 metal-organic phosphorous complex catalyst, and the content ratio of the high boiling point by-product is at least 30% by mass with respect to the catalyst composition that is 100% by mass".

Document 1: WO 2019/098242 A1, document 2: JP 57-122948 A, document 3: JP 2001-114794 A, document 4: JP 2006-151890 A, document 5: JP 55-159841 A, document 6: WO 2005/049202 A1, document 7: JP 8-337550 A

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
☐ The additional search fees were accompanied by the applicant's protest and, where applicable, the payment of a protest fee.
☐ The additional search fees were accompanied by the applicant's protest but the applicable protest fee was not paid within the time limit specified in the invitation.
☐ No protest accompanied the payment of additional search fees.

**INTERNATIONAL SEARCH REPORT**
**Information on patent family members**

International application No.
**PCT/JP2022/044805**

| Patent document cited in search report | Publication date (day/month/year) | Patent family member(s) | Publication date (day/month/year) |
|---|---|---|---|
| WO 2019/098242 A1 | 23 May 2019 | US 2020/0270186 A1<br>claims, paragraph [0082], examples<br>EP 3712126 A1<br>CN 111344273 A | |
| JP 57-122948 A | 31 July 1982 | US 4473655 A<br>claims, 5th column, lines 46-57, examples, reference examples | |
| JP 2001-114794 A | 24 April 2001 | (Family: none) | |
| JP 2006-151890 A | 15 June 2006 | (Family: none) | |
| JP 55-159841 A | 12 December 1980 | EP 17183 A2<br>claims, p. 24, lines 1-15, examples | |
| WO 2005/049202 A1 | 02 June 2005 | CN 1744950 A<br>claims, p. 11, lines 21-29, examples | |
| JP 8-337550 A | 24 December 1996 | US 5648554 A<br>claims, 30th column, lines 49-62, 32th column, lines 13-46 | |
| JP 2013-147445 A | 01 August 2013 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019098242 A
- JP 2006151826 A
- JP S5787845 A
- JP S57122948 A
- JP H6122642 A
- US 3415906 A
- US 4599206 A
- US 4717775 A
- US 4567306 A
- JP S62116535 A
- JP S62116587 A
- JP H5178779 A
- JP H8259578 A
- WO 2019039565 A
- JP 2021202681 A